# EUROPÄISCHE PATENTSCHRIFT

(11) **EP 0 930 868 B1**
(45) Veröffentlichungstag und Bekanntmachung des Hinweises auf die Patenterteilung: **30.07.2003**
(21) Anmeldenummer: 97913111.7
(22) Anmeldetag: 17.10.1997
(51) Int. Cl.: A61K 7/32

(54) **ANTITRANSPIRANT- UND DEODORANTSTIFTE MIT HOHEM WASSERGEHALT**
HIGHLY HYDRATED ANTI-PERSPIRANT AND/OR DEODORIZING STICKS
BATONS ANTISUDORAUX ET/OU DESODORISANTS A FORTE TENEUR EN EAU

(30) Priorität: 19.10.1996 DE 19643238
(43) Veröffentlichungstag der Anmeldung: 28.07.1999
(73) Patentinhaber: Beiersdorf Aktiengesellschaft, 20245 Hamburg (DE)
(72) Erfinder: DIEC, Khiet, Hien, D-22523 Hamburg (DE); KLIER, Manfred, D-21521 Aumühle (DE); SCHREIBER, Jörg, D-22087 Hamburg (DE); WOLF, Florian, D-20251 Hamburg (DE)
(86) Internationale Anmeldenummer: DE9702397
(87) Internationale Veröffentlichungsnummer: WO98017238

(56) Entgegenhaltungen:
- EP-A- 0 522 624
- WO-A-93/04658
- DE-A- 2 335 549
- US-A- 4 725 431
- PATENT ABSTRACTS OF JAPAN vol. 003, no. 075 (C-050), 27.Juni 1979 & JP 54 049337 A (KANEBO LTD), 18.April 1979,
- G. PROSERPIO: "Stabilisation des émulsions" PARFUMS COSMET. AROMES, Nr. 39, 1981, Seiten 71-75, XP002057440
- W.E. ADAM: "Neue Polyalkylenglykol-Copolymere für die Kosmetik" SEIFEN ÖLE FETTE WACHSE, Bd. 110, Nr. 15, 1984, Seiten 427-431, XP002057441
- D. BOUTET: "Nouveaux glycols copolymères utilisés en cosmétique" PARFUMS COSMET. AROMES, Nr. 54, 1983, Seiten 49-53, XP002057442

## Beschreibung

Die vorliegende Erfindung betrifft kosmetische Stifte, welche sich durch einen hohen Wassergehalt auszeichnen, und welche als vorteilhafte Ausführungsformen W/O-Emulsionen darstellen können. Insbesondere betrifft die Erfindung desodorierende oder antitranspirierend wirksame Stifte.

Technisch betrachtet, sind die meisten Stiftformulierungen wasserfreie Fettmischungen aus festen oder halbfesten Wachsen und flüssigen Ölen, wobei die hochgereinigten Paraffinöle und -wachse die Lippenstiftgrundmasse darstellen. Auch wasserhaltige Zubereitungen sind bekannt, welche gelegentlich auch in Form von W/O-Emulsionen vorliegen.

Nach dem idealen Anforderungsprofil sollen sich kosmetische oder pharmazeutische Stifte glatt und ohne großen Reibungswiderstand auftragen lassen. Darüber hinaus muß eine solche Formulierung auch noch die Anforderungen erfüllen, daß der betreffende Stift bruchfest und temperaturbeständig sein muß und die Formulierung nicht ausölen darf.

Von einem Deodorantstift bzw. einem Antitranspirantstift auf der anderen Seite wird erwartet, daß er keinen fettigen Eindruck in der Achsel erzeugt.

Sollen kosmetische oder pharmazeutische Stifte bestimmte Wirkstoffe enthalten, ist denkbar, daß die übrigen Bestandteile mit den Wirkstoffen nicht kompatibel sind. Dies ist besonders häufig der Fall, wenn die Verwendung der kosmetischen Stifte als Deo-Stifte vorgesehen ist, und insbesondere als antitranspirierend wirksame Stifte. Letztere enthalten in der Regel Aluminiumchlorhydrat, welches als kräftige Lewis-Säure gerade für viele Stiftformulierungen nicht verwendbar war. Gerade desodorierende Stifte werden nämlich in der Regel aus Seifen-Glykol-Gelen gebildet, die in dem Umstande begründet liegen, daß niedere Glycole und Glycerin in Gegenwart von Natriumstearat klare, transparente Gele bilden können, welche zusätzlich Alkohol und Wasser aufnehmen können. Solche Formulierungen aber sind nicht mit Aluminiumchlorhydrat verträglich.

Aus Gründen der Verträglichkeit ist es stets zu bevorzugen, selbst bei Verwendung an sich unbedenklicher Substanzen, entsprechende Einsatzkonzentrationen solcher Wirkstoffe möglichst niedrig zu halten.

Kosmetische Desodorantien dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

In sogenannten Antitranspirantien kann durch Adstringentien - vorwiegend Aluminiumsalze wie Aluminiumhydroxychlorid (Aluchlorhydrat) - die Bildung des Schweißes reduziert werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich.

Bekannt und gebräuchlich sind neben den flüssigen Desodorantien auch feste Zubereitungen, beispielsweise Deo-Stifte ("Sticks"), Puder, Pudersprays, Intimreinigungsmittel usw.

Eine weitere Aufgabe der vorliegenden Erfindung war es also, Zubereitungen zu entwikkeln, welche als Grundlage für kosmetische Desodorantien bzw. Antitranspirantien geeignet sind, und die Nachteile des Standes der Technik nicht aufweisen. Weiterhin war es also eine Aufgabe der Erfindung, kosmetische Grundlagen für kosmetische Desodorantien zu entwickeln, die sich durch gute Hautverträglichkeit auszeichnen.

Übliche Grundstoffe des Standes der Technik für stiftförmige Zubereitungen sind beispielsweise flüssige Öle (z.B. Paraffinöle, Ricinusöl, Isopropylmyristat), halbfeste Bestandteile (z.B. Vaseline, Lanolin), feste Bestandteile (z.B. Bienenwachs, Ceresin und mikrokristalline Wachse bzw. Ozokerit) hochschmelzende Wachse (z.B. Carnaubawachs, Candelillawachs).

Der Stand der Technik hat aber eine Reihe von Nachteilen. Dazu zählt die Tatsache, daß wasserlösliche Wirkstoffe häufig nicht gut genug fettlöslich sind, als daß sie in nennenswertem Maße in die kosmetischen Grundlagen einzubauen wären. Andererseits wäre ein gewisser Wassergehalt durchaus erwünscht, um die Kompatibilität des kosmetischen Stiftes mit der menschlichen Haut zu erhöhen. Femer war die Herstellung kosmetischer Stifte mit sehr hohen Wasseranteilen nach dem Stand der Technik erschwert oder unmöglich, weil Wasser mit der hydrophoben Öl/Wachs/Emulgator-Matrix in der Regel nicht kompatibel ist.

Aus dem DBP 23 35 549 ist ein Verfahren zur Herstellung eines kosmetischen Stiftes auf der Basis einer W/O-Emulsion bekannt. Nach dieser Lehre wird aus einer Polyhydroxyverbindung und einer nichtionogenen, oberflächenaktiven Verbindung ein Gel hergestellt, dieses mit einer kosmetischen Grundlage vermischt und Wasser in die Mischung emulgiert.

Nach diesem Verfahren sind jedoch keine Stifte herzustellen, die über die gestellten universellen Anforderungen an einen kosmetischen Stift verfügen. Da dieses Verfahren darüberhinaus kein Ein-Schritt-Verfahren darstelt, zeichnet es sich durch weitere Nachteile aus.

Die DE-OS 41 28 748 beschreibt kosmetische Stifte, welche dadurch gekennzeichnet sind, daß sie Emulsionen darstellen und als wesentliche Bestandteile Bienenwachs, einen oder mehrere Ester aus einer gesättigten Carbonsäure mit 20 - 40 Kohlenstoffatomen und einem gesättigten Alkohol mit 14 - 34 Kohlenstoffatomen, Wasser, sowie gegebenenfalls weitere Lipide und/oder übliche Hilfs- und Zusatzstoffe enthalten. Obwohl diese Zubereitungen zwar vorteilhafte Eigenschaften haben, sind doch noch gewisse Nachteile in Kauf zu nehmen.

Die US-PS 4,719,103 beschreibt einen Antitranspirantstift auf der Basis einer W/O-Emulsion, welcher einen hohen Wasseranteil enthalte, welcher sich auszeichnet durch einen Gehalt an flüchtigen Silikonkomponenten, ein festes Alkanol sowie Polyglycerinfettsäureester, beispielsweise Polyglycerylisostearat, als Emulgator. Die US-PS 4,704,271 und die US-PS 4,725,431 beschreiben ähnliche Zubereitungen.

Die GB-OS 2 162 439 beschreibt paraffinhaltige Stifte, welche einen hohen Wasseranteil enthalten sollen, wobei die Emulgatoren aus der Gruppe der Metallsalze gewählt werden.

Es war nach all diesem überraschend und nicht vorhersehbar, daß Antitranspirant- und/oder Deodorantstifte, dadurch gekennzeichnet, daß sie
(a) eine Fettphase , welche
   (a1) mindestens eine Ölkomponente
   (a2) mindestens eine Wachskomponente
   (a3) gegebenenfalls weitere in der Fettphase lösliche oder dispergierbare Substanzen
   umfaßt:
(b) eine Wasserphase, welche
   (b1) 30 bis 85 Gew.% Wasser bezogen auf das Gesamtgewicht der Stiftfüllgut-Masse sowie
   (b2) gewünschtenfalls in Wasser lösliche oder dispergierbare Substanzen umfaßt,
c) mindestens eine als Antitranspirant und/oder als Deodorant wirkende Substanz in wirksamer Konzentration,
d) mindestens einen W/O-Emulgator oder ein Gemisch aus mehreren W/O-Emulgatoren,
(e) eine oder mehrere Stabilisatoren, gewählt aus der Gruppe der Substanzen der allgemeinen Struktur A-B-A', wobei A und A' gleiche oder verschiedene hydrophobe organische Reste darstellen und B eine hydrophile Gruppe bedeutet,
(f) gewünschtenfalls weitere grenzflächenaktive Substanzen als Coemulgatoren enthalten, ferner gewünschtenfalls weitere Stabilisatoren und weitere übliche kosmetische und/oder pharmazeutische Hilfs-, Wirk- und/oder Zusatzstoffe,
   enthalten,
die Nachteile des Standes der Technik beseitigen.

Es war erstaunlich, daß die erfindungsgemäßen Zubereitungen die Einarbeitung hoher Wassermengen, selbst bei Gegenwart nur geringer Mengen an erfindungsgemäß verwendeten Emulgatoren erlaubt. Die Freisetzung insbesondere wasserlöslicher Wirkstoffe ist gegenüber den herkömmlicher Zubereitungen deutlich erhöht. Ein Beispiel ist die Steigerung der Antitranspiranswirkung von Aluminiumchlorhydrat, welches erfindungsgemäß in geringerer Konzentration besser wirksam ist als die Zubereitungen des Standes der Technik also zum Beispiel im Vergleich zu W/O-Stiften mit niedrigem Wassergehalt oder im Vergleich zu wasserfreien Suspensionstiften, in denen das Aluminiumsalz dispergiert vorliegt. Diese Wirkung läßt sich nochmals steigern, wenn man anstelle des Aluminiumchlorhydrats die Aluminium-Zirkonium-Salze einsetzt.

Femer war überraschend, daß auch die desodorierende Wirkung der erfindungsgemäßen Stifte besser ist als der von Stiften des Standes der Technik.

Aber auch die kosmetischen Eigenschaften der erfindungsgemäßen wasserhaltigen Stifte erweisen sich gegenüber denen des Standes der Technik signifikant verbessert. Beispielsweise läßt sich selbst ohne weitere Zusätze eine angenehme Kühlwirkung auf der Haut durch bloßes Auftragen erzielen, was sich insbesondere bei der Verwendung als Deo-Stift bzw. Antitranspirantstift angenehm bemerkbar macht.

Die Herstellung erfindungsgemäßer Stifte ist dabei sehr einfach, da es sich um ein Ein-Schritt-Verfahren handelt, bei der beispielsweise die Wässerphase zur heißen Fettphase gegeben und anschließend auf Raumtemperatur abgekühlt wird.

Femer zeichnet sich das erfindungsgemäße Verfahren dadurch aus, daß zur Herstellung der erfindungsgemäßen Stifte eine Vielzahl von Emulgatoren beziehungsweise Ölkomponenten eingesetzt werden können.

Es ist erfindungsgemäß vorteilhaft, wenngleich nicht zwingend, daß die erfindungsgemäßen Zubereitungen im physikalisch-chemischen Sinne eine W/O-Emulsion darstellen. Es sind indes auch andere Erscheinungsformen der erfindungsgemäßen Zubereitungen möglich und gegebenenfalls vorteilhaft.

Der erfindungsgemäß verwendete W/O-Emulgator bzw. die W/O-Emulgatoren aus der Substanzgruppe A-B-A' wird oder werden erfindungsgemäß vorteilhaft gewählt aus der Gruppe der Substanzen der allgemeinen Formel 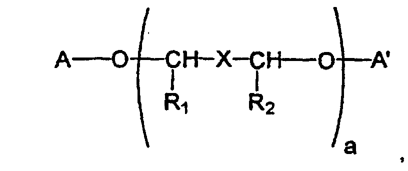 wobei
- A und A' gleiche oder verschiedene hydrophobe organische Reste darstellen,
- a eine Zahl von 1 bis 100, vorzugsweise 2 bis 60, insbesondere 5 bis 40 darstellt,
- X eine Einfachbindung oder die Gruppe 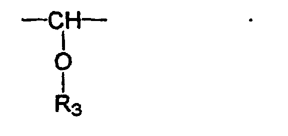 darstellt,
- R₁ und R₂ unabhängig voneinander aus der Gruppe H, Methyl gewählt werden daß aber nicht beide Reste gleichzeitig Methyl darstellen,
- R₃ gewählt wird aus der Gruppe H, sowie der verzweigten und unverzweigten, gesättigten und ungesättigten Alkyl- und Acylreste mit 1 - 20 Kohlenstoffatomen.

Die Strukturformel ist nicht so zu interpretieren, daß durch den Index a alle in der Klammer repräsentierten Reste R₁, R₂ bzw R₃ im gesamten Molekül jeweils gleich sein müssen. Vielmehr können diese Reste in jedem der a Fragmente 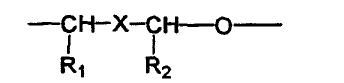 frei gewählt werden

Die Reste A und A' werden vorteilhaft gewählt aus der Gruppe der verzweigten und unverzweigten, gesättigten und ungesättigten Alkyl- und Acylreste und Hydroxyacylreste mit 10 - 30 Kohlenstoffatomen sowie femer aus der Gruppe der über Esterfunktionen miteinander verbundenen Hydroxyacylgruppen, nach dem Schema 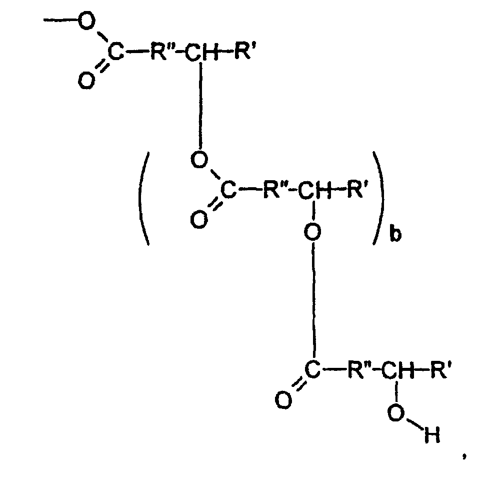 wobei R' gewählt wird aus der Gruppe der verzweigten und unverzweigten Alkylgruppen mit 1 bis 20 Kohlenstoffatomen und R" gewählt wird aus der Gruppe der verzweigten und unverzweigten Alkylengruppen mit 1 bis 20 Kohlenstoffatomen und b Zahlen von 0 bis 200 annehmen kann.

Beispiele für besonders vorteilhaft im Sinne der vorliegenden Erfindung zu verwendende W/O-Emulgatoren des A-B-A'-Typs sind PEG-30 Dipolyhydroxystearat, Decaglycerylheptaoleat, Polyglyceryl-3-Diisostearat, PEG-8-Distearat, Diglycerindipolyhydroxystearat.

Erfindungsgemäß können der oder die W/O-Emulgatoren allerdings auch gewählt werden aus der Gruppe Fettalkohole mit 8 - 30 Kohlenstoffatomen, Monoglycerinester gesättigter und/oder ungesättigter, verzweigter und/oder unverzweigter Alkancarbonsäuren einer Kettenlänge von 8 - 24, insbesondere 12 - 18 C-Atomen, Diglycerinester gesättigter und/oder ungesättigter, verzweigter und/oder unverzweigter Alkancarbonsäuren einer Kettenlänge von 8 - 24, insbesondere 12 - 18 C-Atomen, Triglycerinester gesättigter und/oder ungesättigter, verzweigter und/oder unverzweigter Alkancarbonsäuren einer Kettenlänge von 8 - 24, insbesondere 12 - 18 C-Atomen, Polyglycerinester gesättigter und/oder ungesättigter, verzweigter und/oder unverzweigter Alkancarbonsäuren einer Kettenlänge von 8 - 24, insbesondere 12 - 18 C-Atomen mit bis zu 10 Glycerineinheiten, Monoglycerinether gesättigter und/oder ungesättigter, verzweigter und/oder unverzweigter Alkohole einer Kettenlänge von 8 - 24, insbesondere 12 -18 C-Atomen, Diglycerinether gesättigter und/oder ungesättigter, verzweigter und/oder unverzweigter Alkohole einer Kettenlänge von 8 - 24, insbesondere 12 - 18 C-Atomen, Triglycerinether gesättigter und/oder ungesättigter, verzweigter und/oder unverzweigter Alkohole einer Kettenlänge von 8 - 24, insbesondere 12 - 18 C-Atomen, Polyglycerinether gesättigter und/oder ungesättigter, verzweigter und/oder unverzweigter Alkancarbonsäuren einer Kettenlänge von 8 - 24, insbesondere 12 - 18 C-Atomen mit bis zu 10 Glycerineinheiten, Propylenglycolester gesättigter und/oder ungesättigter, verzweigter und/oder unverzweigter Alkancarbonsäuren einer Kettenlänge von 8 - 24, insbesondere 12 - 18 C-Atomen, Sorbitanester gesättigter und/oder ungesättigter, verzweigter und/oder unverzweigter Alkancarbonsäuren einer Kettenlänge von 8 - 24, insbesondere 12 - 18 C-Atomen, Sorbitanester von Polyolen, insbesondere des Glycerins, Pentaerythritylester gesättigter und/oder ungesättigter, verzweigter und/oder unverzweigter Alkancarbonsäuren einer Kettenlänge von 8 - 24, insbesondere 12 - 18 C-Atomen, Methylglucose Ester gesättigter und/oder ungesättigter, verzweigter und/oder unverzweigter Alkancarbonsäuren einer Kettenlänge von 8 - 24, insbesondere 12 - 18 C-Atomen, Polyglycerin Methylglucose Ester gesättigter und/oder ungesättigter, verzweigter und/oder unverzweigter Alkancarbonsäuren einer Kettenlänge von 8 - 24, insbesondere 12 - 18 C-Atomen.

Es kann erfindungsgemäß von Vorteil sein, daß die vorstehend genannten Typen von W/O-Emulgatoren zusätzlich polyethoxyliert und/oder polypropoxyliert sind, oder daß auch andere polyethoxylierte und/oder polypropoxylierte Produkte Verwendung finden, beispielsweise polyethoxyliertes hydrogeniertes oder nichthydrogeniertes Ricinusöl, ethoxyliertes Cholesterin.

Insbesondere vorteilhafte W/O-Emulgatoren sind Glyceryllanolat, Glycerylmonostearat, Glycerylmonoisostearat, Glycerylmonomyristat, Glycerylmonooleat, Diglycerylmonostearat, Diglycerylmonoisostearat, Diglyceryldiisostearat, Propylenglycolmonostearat, Propylenglycolmonoisostearat, Propylenglycoldiisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, 2-Ethylhexylglycerinether, Selachylalkohol, Chimylalkohol, Polyethylenglycol(2)stearylether (Steareth-2), Glycerylmonolaurat, Glycerylmonocaprinat, Glycerylmonocaprylat, Glycerylsorbitanstearat Polyglyceryl-4-Isostearat, Polyglyceryl-2-sesquiisostearat, PEG-7 hydrogeniertes Ricinusöl, PEG-40-Sorbitanperisostearat, Isostearyldiglycerylsuccinat, PEG-5-Cholesterylether.

Der erfindungsgemäß verwendete W/O-Emulgator bzw. die erfindungsgemäß verwendeten W/O-Emulgatoren, welcher oder welche in das Schema A-B-A' passen, liegt bzw. liegen vorteilhaft in Konzentrationen von 0,1 - 25 Gew.-% vor, wobei es allerdings möglich und vorteilhaft ist, den Gehalt an Emulgatoren niedrig zu halten, etwa bis 5 Gew.-%, jeweils bezogen auf das Gesamtgewicht der Zusammensetzung. Es ist vorteilhaft, die Gesamtkonzentration der W/O-Emulgatoren, was auch diejenigen Emulgatoren einschließt, die nicht in das Schema A-B-A' passen, nicht größer als etwa 25 - 30 Gew.-% und nicht geringer als etwa 0,1 Gew.-% zu wählen, jeweils bezogen auf das Gesamtgewicht der Zubereitungen.

Die erfindungsgemäß verwendeten Stabilisatoren werden erfindungsgemäß vorteilhaft gewählt aus der Gruppe der Substanzen der allgemeinen Formel 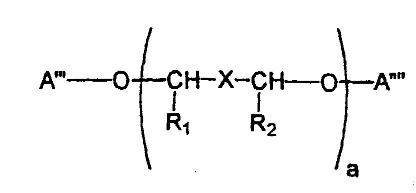 ,wobei
- A"' und A"" gleiche oder verschiedene hydrophobe organische Reste darstellen,
- a eine Zahl von 1 bis 100, vorzugsweise 2 bis 60, darstellt,
- X eine Einfachbindung oder die Gruppe 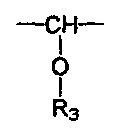
- darstellt,
- R₁ und R₂ unabhängig voneinander aus der Gruppe H, Methyl gewählt werden daß aber nicht beide Reste gleichzeitig Methyl darstellen,
- R₃ gewählt wird aus der Gruppe H, sowie der verzweigten und unverzweigten, gesättigten und ungesättigten Alkyl- und Acylreste mit 1- 20 Kohlenstoffatomen.

Die Strukturformel ist nicht so zu interpretieren, daß durch den Index a alle in der Klammer repräsentierten Reste R₁, R₂ bzw R₃ im gesamten Molekül jeweils gleich sein müssen. Vielmehr können diese Reste in jedem der a Fragmente 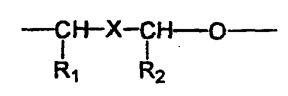 frei gewählt werden

Die Reste A"' und A"" können gleich oder verschieden sein und werden bevorzugt gewählt aus der Gruppe 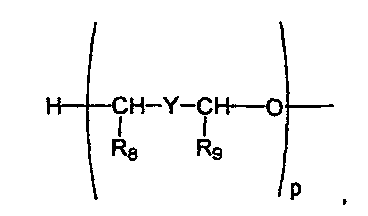 wobei R₈ und R₉ gleich oder verschieden sein können und gewählt werden aus der Gruppe der gesättigten und ungesättigten Alkyl- und Acylreste mit 1 - 30 Kohlenstoffatomen, p eine Zahl von 1 - 20 darstellt und Y eine Einfachbindung oder die Gruppe 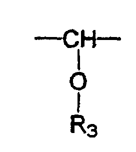 darstellt, wobei R₃ gewählt wird aus der Gruppe H, sowie der verzweigten und unverzweigten, gesättigten und ungesättigten Alkyl- und Acylreste mit 1 - 30 Kohlenstoffatomen.

Bevorzugter Stabilisator ist das PEG-45 /Dodecylglycolcopolymer, welches die Struktur 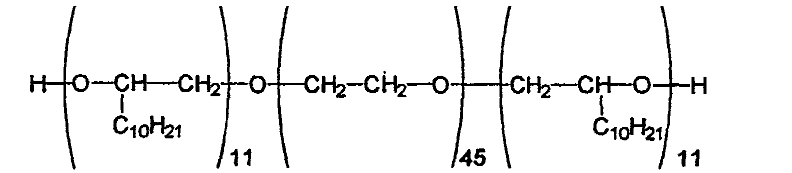 besitzt. Es wird von der Gesellschaft Akzo Nobel Chemicals GmbH unter der Bezeichnung ELFACOS® ST 9 angeboten. Aber auch das entsprechende PEG-22 / Dodecylglycolcopolymer ist vorteilhaft zu verwenden.

Femer können die Gruppe A"' und A"" unabhängig voneinander auch Alkylreste oder Acylreste darstellen. Besonders vorteilhaft ist auch als Stabilisator das Methoxy PEG-22/Dodecyl Glycol Copolymer zu verwenden. Es wird von der Gesellschaft Akzo Nobel Chemicals GmbH unter der Bezeichnung ELFACOS® E 200 angeboten.

Der Stabilisator bzw. die Stabilisatoren liegen vorteilhaft in Konzentrationen von 0,01 - 25 Gew.-% vor, wobei es allerdings möglich und vorteilhaft ist, den Gehalt an Stabilisatoren niedrig zu halten, etwa bis 5 Gew.-%, jeweils bezogen auf das Gesamtgewicht der Zusammensetzung.

Es ist insbesondere dann vorteilhaft, Stabilisatoren zu wählen, wenn erfindungsgemäße Zubereitungen einen hohen Gehalt an destabilisierenden Substanzen, beispielsweise Lewis-Säuren, und insbesondere Aluchlorhydrat enthalten sollen. Ist der Gehalt an destabilisierenden Substanzen gering, kann man auf den Stabilisator verzichten.

Die Ölkomponente oder die Gesamtheit der Ölkomponenten der erfindungsgemäßen wasserhaltigen, kosmetischen Stifte sollen bei Raumtemperatur eine Flüssigkeit darstellen, die Wachskomponente oder die Gesamtheit der Wachskomponente sollen bei Raumtemperatur einen Festkörper bilden. Es ist von Vorteil, die Ölkomponenten und die Wachskomponenten so aufeinander abzustimmen, daß das Gemisch aus Ölkomponenten und Wachskomponenten ohne restliche Komponenten, also etwa ohne Wasserphase und ohne Emulgator, bei Raumtemperatur einen Festkörper bildet.

Die Ölkomponente oder die Gesamtheit der Ölkomponenten der erfindungsgemäßen wasserhaltigen, kosmetischen Stifte wird bevorzugt gewählt aus der Gruppe der Ester aus gesättigten und/oder ungesättigten, verzweigten und/oder unverzweigten Alkancarbonsäuren einer Kettenlänge von 1 bis 44 C-Atomen und gesättigten und/oder ungesättigten, verzweigten und/oder unverzweigten Alkoholen einer Kettenlänge von 1 bis 44 C-Atomen, aus der Gruppe der Ester aus aromatischen Carbonsäuren und gesättigten und/oder ungesättigten, verzweigten und/oder unverzweigten Alkoholen einer Kettenlänge von 1 bis 30 C-Atomen sofern die Ölkomponente oder die Gesamtheit der Ölkomponenten bei Raumtemperatur eine Flüssigkeit darstellen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Femer kann die Ölphase vorteilhaft gewählt werden aus der Gruppe der verzweigten und unverzweigten Kohlenwasserstoffe, der Silkonöle, der Dialkylether, der Gruppe der gesättigten oder ungesättigten, verzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Vorteilhaft wird die Ölphase ferner gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether, Ethylenglycoldioleat, Di-(2-Ethylhexyl)adipat).

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Bevorzugt ist, die Ölkomponenten zu wählen aus der Gruppe der
- Ester aus gesättigten verzweigten Alkancarbonsäuren einer Kettenlänge von 1 bis 44 C-Atomen und gesättigten unverzweigten Alkoholen einer Kettenlänge von 1 bis 44 C-Atomen, sofern die Ölkomponente oder die Gesamtheit der Ölkomponenten bei Raumtemperatur eine Flüssigkeit darstellen,
und/oder der
- Ester aus gesättigten unverzweigten Alkancarbonsäuren einer Kettenlänge von 1 bis 44 C-Atomen und gesättigten verzweigten Alkoholen einer Kettenlänge von 1 bis 44 C-Atomen, sofern die Ölkomponente oder die Gesamtheit der Ölkomponenten bei Raumtemperatur eine Flüssigkeit darstellen.

Besonders bevorzugt ist, die Ölkomponenten zu wählen aus der Gruppe der
- Ester aus gesättigten verzweigten Alkancarbonsäuren einer Kettenlänge von 14 bis 44 C-Atomen und gesättigten verzweigten Alkoholen einer Kettenlänge von 14 bis 44 C-Atomen, sofern die Ölkomponente oder die Gesamtheit der Ölkomponenten bei Raumtemperatur eine Flüssigkeit darstellen.

Besonders vorteilhafte Ölkomponenten können aus der Gruppe Isotridecylisononanoat, Isocetylstearat. Isopropylstearat, Isopropylisostearat, Butyloctansäure-2-butyloctanoat, 2-Ethylhexylisostearat (=Octylisostearat) Cetearylisononanoat, C₁₂-C₁₅-Alkylbenzoat, C₁₂-C₁₅-Alkohollactat, Glyceryltriisostearin, Cyclomethicon, Isohexadecan, gewählt werden.

Die Ölkomponenten können vorteilhaft in einem Gehalt von 0,5 bis 80 Gew.-%, bezogen auf die Gesamtzubereitung vorliegen, bevorzugt sind etwa 1 bis 20 Gew.-%.

Die Wachskomponente oder die Gesamtheit der Wachskomponenten der erfindungsgemäßen wasserhaltigen, kosmetischen Stifte wird bevorzugt gewählt aus der Gruppe der Ester aus gesättigten und/oder ungesättigten, verzweigten und/oder unverzweigten Alkancarbonsäuren einer Kettenlänge von 1 bis 80 C-Atomen und gesättigten und/oder ungesättigten, verzweigten und/oder unverzweigten Alkoholen einer Kettenlänge von 1 bis 80 C-Atomen, aus der Gruppe der Ester aus aromatischen Carbonsäuren bzw. Hydroxycarbonsäuren (z.B. 12-Hydroxystearinsäure) und gesättigten und/oder ungesättigten, verzweigten und/oder unverzweigten Alkoholen einer Kettenlänge von 1 bis 80 C-Atomen sofern die Wachskomponente oder die Gesamtheit der Wachskomponenten bei Raumtemperatur einen Festkörper darstellen.

Bevorzugt ist, die Wachskomponenten zu wählen aus der Gruppe der
- Ester aus gesättigten verzweigten Alkancarbonsäuren einer Kettenlänge von 1 bis 60 C-Atomen und gesättigten unverzweigten Alkoholen einer Kettenlänge von 1 bis 60 C-Atomen, sofern die Wachskomponente oder die Gesamtheit der Wachskomponenten bei Raumtemperatur einen Festkörper darstellen,
und/oder der
- Ester aus gesättigten unverzweigten Alkancarbonsäuren einer Kettenlänge von 1 bis 60 C-Atomen und gesättigten verzweigten Alkoholen einer Kettenlänge von 1 bis 60 C-Atomen, sofern die Wachskomponente oder die Gesamtheit der Wachskomponenten bei Raumtemperatur einen Festkörper darstellen.

Besonders bevorzugt ist, die Wachskomponenten zu wählen aus der Gruppe der
- Ester aus gesättigten verzweigten Alkancarbonsäuren einer Kettenlänge von 14 bis 44 C-Atomen und gesättigten verzweigten Alkoholen einer Kettenlänge von 14 bis 44 C-Atomen, sofern die Wachskomponente oder die Gesamtheit der Wachskomponenten bei Raumtemperatur einen Festkörper darstellen.

Insbesondere vorteilhaft können die Wachskomponenten aus der Gruppe der C₁₆₋₃₈-Alkylstearate, der C₁₀₋₄₀-Alkylstearate, der C₂₀₋₄₀Alkylisostearate, der C₂₀₋₄₀-Dialkyldimerate, der C₁₈₋₃₈ Alkylhydroxystearoylstearate, der C₂₀₋₄₀-Alkylerucate gewählt werden, ferner C₃₀₋₅₀-Alkylbienenwachs, Cetearylbehenat. Auch Siliconwachse wie beispielsweise Stearyltrimethylsilan/Stearylalkohol sind gegebenenfalls vorteilhaft.

Insbesondere pflanzliche und/oder tierische Wachse oder chemisch modifizierte Derivate davon, insbesondere Carnaubawachs, Candelillawachs, Sonnenblumenwachs, Reiswachse, Fruchtwachse wie Orangenwachs, Zitronenwachs, Grapefruitwachs, Lorbeerwachs (= Bayberrywax) und dergleichen, sind vorteilhaft zu verwenden. Außerdem können diese natürlichen Wachse auch ohne synthetische Wachse allein eingesetzt werden.

Die Wachskomponenten können vorteilhaft in einem Gehalt von 0,5 bis 80 Gew.-%, bezogen auf die Gesamtzubereitung vorliegen, bevorzugt sind etwa 1 bis 20 Gew.-%.

Es ist von Vorteil, das Verhältnis von Öl- und Wachskomponenten zueinander ungefähr aus dem Bereich der Gewichtsverhältnisse zwischen 2 : 1 bis 1 : 2, insbesondere 3 : 2 bis 2 : 3, ganz besonders bevorzugt ca. 1 : 1, einzustellen.

Die Wassermenge kann bis zu etwa 85 Gew.-% betragen, bezogen auf das Gesamtgewicht der Zubereitungen, wobei üblicherweise optimale Wassergehalte im Bereich zwischen 50 und 75 Gew.-% gewählt werden. Gewünschtenfalls kann der Mindestwassergehalt zwar 10 Gew.-% unterschreiten. Es ist aber von größerem Vorteil, erfindungsgemäße Stifte mit Gehalt von über 10 Gew.-% Wasser auszustatten.

Es ist auch möglich, zusätzliche Substanzen zu verwenden, welche die Konsistenz der erfindungsgemäßen Zubereitungen modifizieren, beispielsweise Verdicker, welche gewählt werden können aus der Gruppe der Substanzen welche mindestens zwei hydrophile Reste tragen, welche über eine hydrophobe Gruppierung miteinander verbunden sind, also den Molekülschemata 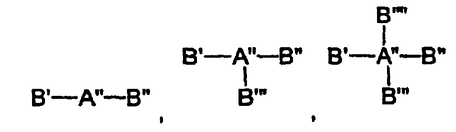 usw.
folgen.
Dabei stellen die Reste B mit den verschiedenen Indizes hydrophile Gruppen dar, die Reste A mit den verschiedenen Indizes hydrophobe Gruppen.

Solche Verdicker werden bevorzugt gewählt aus der Gruppe der Triblockcopolymere des Typs 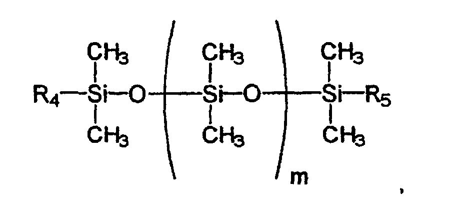 wobei m eine Zahl von 10 bis 10000 darstellen kann, R₄ und R₅ gleich oder verschieden sein können und gewählt werden aus der Gruppe, die durch die allgemeine Struktur 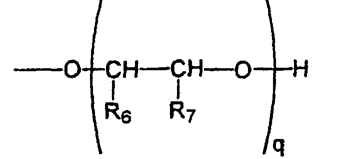 repräsentiert wird. Dabei können R₆ und R₇ unabhängig voneinander so gewählt werden daß sie H und Methyl, daß aber nicht beide Reste gleichzeitig Methyl darstellen können. q ist eine Zahl von 2 bis 1000, bevorzugt von 10 bis 200.

R₄ und R₅ können auch Polylolreste darstellen (z.B. Glyceryl-, Polyglyceryl-, Sorbityl-, Cellulosereste usw.)

In die erfindungsgemäßen Zubereitungen können vorteilhaft zusätzlich die üblichen Bestandteile kosmetischer Stifte eingearbeitet werden, z.B. Kohlenwasserstoffe, Fette und Öle für die Grundsubstanz, sowie die üblichen Hilfs- und Zusatzstoffe wie Parfümöle, Konservierungsmittel, Farbpigmente, Lichtschutzmittel, Stabilisatoren.

Zusätzlich können Pflegewirkstoffe eingearbeitet werden, welche sich nicht wie bisher auf die fettlöslichen Wirkstoffe beschränken, sondern auch aus der Gruppe der wasserlöslichen Wirkstoffe gewählt werden können, beispielsweise Vitamine und dergleichen mehr.

Alle für Desodorantien/Antitranspirantien gängigen Wirkstoffe können vorteilhaft genutzt werden, beispielsweise Geruchsüberdecker wie die gängigen Parfümbestandteile, Geruchsabsorber, beispielsweise die in der Patentoffenlegungsschrift DE-P 40 09 347 beschriebenen Schichtsilikate, von diesen insbesondere Montmorillonit, Kaolinit, llit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen W/O-Emulsionsstifte eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatriën-1-ol) sowie die in den Patentoffenlegungsschriften DE-37 40 186, DE-39 38 140, DE-42 04 321, DE-42 29 707, DE-42 29 737, DE-42 37 081, DE-43 09 372, DE-43 24 219 beschriebenen wirksamen Agenzien. Auch Natriumhydrogencarbonat ist vorteilhaft zu verwenden.

Die üblichen Antitranspiranswirkstoffe können ebenfalls vorteilhaft in den erfindungsgemäßen wasserhaltigen, kosmetischen Stifte verwendet werden, insbesondere Adstringentien, beispielsweise basische Aluminiumchloride.

Die erfindungsgemäßen kosmetischen Desodorantien können in Form von aus normalen Behältern auftragbaren wasserhaltigen, kosmetischen Stifte vorliegen.

Die Menge der Antitranspiranswirkstoffe oder Desodorantien (eine oder mehrere Verbindungen) in den Zubereitungen beträgt vorzugsweise 0,01 bis 30 Gew.-%, besonders bevorzugt 0,1 - 20 Gew.-%, insbesondere 1 - 10 Gew.-%, bezogen auf das Gesamtgewicht der Zubereitung.

Vorteilhafte Wirkstoffe sind weiterhin Antioxidantien, insbesondere solche, welche nicht nur die Bestandteile der Formulierung, sondern auch die Haut vor oxidativer Beanspruchung schützen können.

Die Zubereitungen enthalten daher vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Besonders vorteilhaft werden die Antioxidantien gewählt aus der Gruppe, bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioglycerin, Thiosorbitol, Thioglycolsäure, Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Pentat-, Hexa-, Heptahioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol deren Derivate, Vitamin C und Derivate (z.B. Ascorbylpalmitate, Mg - Ascorbylphosphate, Ascorbylacetate), Isoascorbinsäure und ihre Derivate, Tocopherole und Derivate (z.B. Vitamin E - acetat), Vitamin A und Derivate (Vitamin A - palmitat) sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können zwar öllösliche oder öldispergierbare Antioxidantien eingesetzt werden. Es hat sich jedoch herausgestellt, daß die Erfindung gerade dem Einsatz wasserlöslicher oder wasserdispergierbarer Antioxidantien in Stiftformulierungen die Pforten öffnet.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist es vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 -10 Gew.-%, bezogen auf das Gesamtgewicht der Formulierung, zu wählen.

Es ist dem Fachmanne natürlich bekannt, daß anspruchsvolle kosmetische Zubereitungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Insbesondere dann, wenn die erfindungsgemäßen Zubereitungen sich durch leichte oder erleichterte Abwaschbarkeit von menschlicher Haut auszeichnen sollen, ist es von Vorteil, den Zubereitungen wasserlösliche und/oder mit Wasser quellbare Polymere einzuverleiben, insbesondere mit Alkylgruppen veretherte Cellulose- und/oder Stärkederivate. Besonders vorteilhaft sind β-Glucane, Xanthangummi, Dextrane, Hydroxymethylcellulose, Hydroxyethylcellulose und/oder Hydroxypropylcellulose, Methoxy-PEG-22/Dodecyl-Glycol-Copolymere, Poloxamere.

Vorteilhafte wasserlösliche und/oder mit Wasser quellbare Polymere können auch gewählt werden als mit einem oder mehreren n-Octenylsuccinatresten veresterter hydrophiler Stärke enthalten. Solche Stärkederivate zeichnen sich aus durch eine Struktur

Stärke-Xₙ, wobei X den Rest 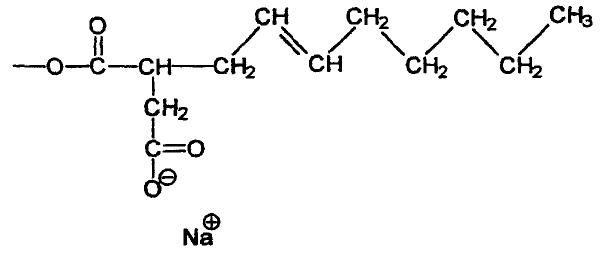 symbolisiert.

Erfindungsgemäß vorteilhaft zu verwendende Stärkederivate tragen offiziell noch keinen INCl-Namen (International Nomenclature Cosmetic Ingredient) dieser müßte die Bezeichnung "Starch Sodium Octenyl Succinate" tragen. Besonders vorteilhaft sind solche Produkte, welcher unter der Bezeichnung Amiogum®, insbesondere Amiogum®23 von der Gesellschaft Cerestar US verkauft werden.

Es wird bevorzugt, den Gehalt an wasserlöslichen und/oder mit Wasser quellbaren Polymeren im Konzentrationsbereich von 0,01-5,0 Gew.-%, besonders bevorzugt 0,1 - 1,0 Gew.-%, zu wählen.

Es hat sich in erstaunlicher Weise herausgestellt, daß die erfindungsgemäß verwendeten wasserlöslichen und/oder mit Wasser quellbaren Polymere darüberhinaus die Hautfreundlichkeit der erfindungsgemäßen kosmetischen Zubereitungen erhöhen. Es wird ein angenehmeres Gefühl beim Auftragen der Stiftmasse auf die Haut erzielt.

Die Einarbeitung solcher wasserlöslichen und/oder mit Wasser quellbaren Polymere erfolgt bevorzugt dadurch, daß sie der Wasserphase einverleibt und mit der Wasserphase, besonders bevorzugt nach vollständiger Auflösung bzw. Quellung in die aufgeschmolzene Fettphase der Zubereitungen gegeben werden.

Erfindungsgemäß können Wirkstoffe auch sehr vorteilhaft gewählt werden aus der Gruppe der lipophilen Wirkstoffe, insbesondere aus folgender Gruppe:

Acetylsalicylsäure, Atropin, Azulen, Hydrocortison und dessen Derivaten, z.B. Hydrocortison-17-vaierat, Vitamine, z.B. Ascorbinsäure und deren Derivate, Vitamine der B- und D-Reihe, sehr günstig das Vitamin B₁, das Vitamin B₁₂ das Vitamin D₁, aber auch Bisabolol, ungesättigte Fettsäuren, namentlich die essentiellen Fettsäuren (oft auch Vitamin F genannt), insbesondere die γ-Linolensäure, Ölsäure, Eicosapentaënsäure, Docosahexaënsäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkernöl, Fischöle, Lebertran aber auch Ceramide und ceramidähnliche Verbindungen und so weiter.

Vorteilhaft ist es auch, die Wirkstoffe aus der Gruppe der rückfettenden Substanzen zu wählen, beispielsweise Purcellinöl®, Eucerit® und Neocerit®.

Die erfindungsgemäßen wasserhaltigen, kosmetischen Stifte tragen ferner in vorzüglicher Weise zur Hautglättung bei, insbesondere, wenn sie mit einer oder mehreren Substanzen versehen sind, die die Hautglättung fördern.

Es ist gegebenenfalls möglich und vorteilhaft, die erfindungsgemäßen Zubereitungen als Grundlage für pharmazeutische Formulierungen zu verwenden. Mutatis mutandis gelten entsprechende Anforderungen an die Formulierung medizinischer Zubereitungen. Die Übergänge zwischen reinen Kosmetika und reinen Pharmaka sind dabei fließend. Als pharmazeutische Wirkstoffe sind erfindungsgemäß grundsätzlich alle Wirkstoffklassen geeginet, wobei lipophile Wirkstoffe bevorzugt sind. Beispiele sind: Antihistaminika, Antiphlogistika, Antibiotika, Antimykotika, die Durchblutung fördernde Wirkstoffe, Keratolytika, Hormone, Steroide, Vitamine usw.

Alle Mengenangaben, Prozentangaben oder Teile beziehen sich, soweit nicht anders angegeben, auf das Gewicht, insbesondere auf das Gesamtgewicht der Zubereitungen oder der jeweiligen Mischung.

Die nachfolgenden Beispiele sollen die Erfindung erläutern, ohne sie einzuschränken. Das verwendete Aluminiumchlorhydrat stellt eine 50%ige Lösung dar.

### Beispiel 1

| **Deo/AT-Stift mit hohem Wassergehalt** | |
|---|---|
| | Gew.-% |
| PEG-45/ Dodecyl Glycol Copolymer | 0,800 |
| Polyglyceryl-3-Diisostearat | 1,600 |
| Capryl-/Caprinsäuretriglycerid | 3,000 |
| Octyldodecanol | 4,000 |
| Dicaprylylether | 4,000 |
| C₂₀₋₄₀-Alkylstearate | 12,000 |
| Butandiol | 4,000 |
| Aluminiumchlorhydrat | 20,000 |
| Wasser | ad 100,000 |

### Beispiel 1a

| Deo/AT-Stift mit hohem Wassergehalt und verbessertem Abwaschverhalten | |
|---|---|
| | Gew.-% |
| PEG-45/ Dodecyl Glycol Copolymer | 0,800 |
| Polyglyceryl-3-Diisostearat | 0,800 |
| Methoxy-PEG-22/Dodecyl-Glycol-Copolymere | 0,800 |
| Capryl-/Caprinsäuretriglycerid | 4,000 |
| Octyldodecanol | 4,000 |
| Dicaprylylether | 4,000 |
| C₂₀₋₄₀-Alkylstearate | 12,000 |
| Butandiol | 4,000 |
| Aluminiumchlorhydrat | 20,000 |
| Wasser | ad 100,000 |

### Beispiel 1b

| Deo/AT-Stift mit hohem Wassergehalt und verbessertem Abwaschverhalten | |
|---|---|
| | Gew.-% |
| PEG-45/ Dodecyl Glycol Copolymer | 0,800 |
| Polyglyceryl-3-Diisostearat | 1,600 |
| Hydroxyethylcellulose | 0,400 |
| Capryl-/Caprinsäuretriglycerid | 4,000 |
| Octyldodecanol | 3,000 |
| Dicaprylylether | 4,000 |
| C₂₀₋₄₀-Alkylstearate | 10,000 |
| Butyleneglycoliol | 4,000 |
| Aluminiumchlorhydrat | 20,000 |
| Wasser | ad 100,000 |

### Beispiel 2

| Aminosäurehaltiger-Stift mit hohem Wassergehalt | |
|---|---|
| | Gew.-% |
| Polyglyceryl-3-Diisostearat | 1,801 |
| Wollwachssäure | 0,600 |
| Octylisostearat | 14,006 |
| C₂₀₋₄₀-Alkylstearat | 13,005 |
| PEG-40 Hydriertes Rizinusöl | 0,600 |
| Dipropylenglycol | 1,801 |
| C₁₂₋₁₅ Alcohol-Lactat | 8,804 |
| Glycin | 1,721 |
| Glycerycaprylat | 0,570 |
| Parfum, Konservierungsmittel | q.s. |
| Wasser | ad 100,000 |

### Beispiel 2b

| Aminosäurehaltiger-Stift mit hohem Wassergehalt und verbessertem Abwaschverhalten | |
|---|---|
| | Gew.-% |
| Polyglyceryl-3-Diisostearat | 1,800 |
| Wollwachssäure | 0,600 |
| Xanthangummi | 0,400 |
| Octylisostearat | 14,000 |
| C₂₀₋₄₀-Alkylstearat | 13,000 |
| PEG-40 Hydriertes Rizinusöl | 0,600 |
| Dipropylenglycol | 1,800 |
| C₁₂₋₁₅ Alcohol-Lactat | 8,800 |
| Glycin | 1,720 |
| Glycerycaprylat | 0,570 |
| Parfum, Konservierungsmittel | q.s. |
| Wasser | ad 100,000 |

### Beispiel 3

| Hautfeuchtemittel enthaltender Stift mit hohem Wassergehalt | |
|---|---|
| | Gew.-% |
| Polyglyceryl-3-Diisostearate | 1,801 |
| Wollwachssäure | 0,600 |
| Octylisostearat | 14,006 |
| C₂₀₋₄₀-Alkylstearat | 13,005 |
| PEG-40 Hydriertes Rizinusöl | 0,600 |
| Dipropylene Glycol | 1,801 |
| Glycereth-7 | 8,804 |
| Glycin | 1,721 |
| Glycerylcaprylat | 0,570 |
| Parfum, Konservierungsmittel | q.s. |
| Wasser | ad 100,000 |

### Beispiel 4

| Deo-Stift mit hohem Wassergehalt | |
|---|---|
| | Gew.-% |
| Wollwachssäure | 0,680 |
| PEG-30 Dipolyhydroxystearat | 2,041 |
| Tridecylisononanoat | 25,859 |
| C₂₀₋₄₀-Alkylstearat | 14,744 |
| Dipropylenglycol | 2,041 |
| Aluminiumchlorhydrate | 4,083 |
| Glycerylcaprylat | 0,646 |
| Parfum, Konservierungsmittel | q.s. |
| Wasser | ad 100,000 |

### Beispiel 5

| Deo/AT-Stift mit hohem Wassergehalt | |
|---|---|
| | Gew.-% |
| PEG-45/ Dodecyl Glycol Copolymer | 0,900 |
| Wollwachssäure | 0,600 |
| Decaglycerylheptaoleat | 1,800 |
| Cetylstearylisononanoat | 7,750 |
| Tridecylisononanoat | 7,750 |
| C₂₀₋₄₀-Alkylstearat | 14,500 |
| Dipropylenglycol | 1,800 |
| Aluminiumchlorhydrat | 10,000 |
| Glycerylcaprylat | 0,570 |
| Parfum, Konservierungsmittel | q.s. |
| Wasser | ad 100,000 |

### Beispiel 6

| Deo/AT-Stift mit hohem Wassergehalt | |
|---|---|
| | Gew.-% |
| Polyglyceryl-3-Diisostearat | 2,127 |
| Wollwachssäure | 0,709 |
| Isohexadecan | 2,694 |
| Octylisostearat | 24,244 |
| C₂₀₋₄₀-Alkylstearat | 15,359 |
| PEG-40 Hydriertes Rizinusöl | 0,709 |
| Dipropylenglycol | 2,127 |
| Glycerylcaprylat | 0,673 |
| Parfum, Konservierungsmittel | q.s. |
| Wasser | ad 100,000 |

### Beispiel 7

| Deo-Stift mit hohem Wassergehalt | |
|---|---|
| | Gew.-% |
| PEG-45/ Dodecyl Glycol Copolymer | 0,900 |
| Polyglyceryl-3-Diisostearat | 1,800 |
| Tridecylisononanoat | 15,500 |
| C₂₀₋₄₀-Alkylstearat | 14,500 |
| Dipropylenglycol | 1,800 |
| Glycerinmonolaurat | 2.000 |
| Parfum, Konservierungsmittel | q.s. |
| Wasser | ad 100,000 |

### Beispiel 8

| Deo-Stift mit hohem Wassergehalt | |
|---|---|
| | Gew.-% |
| PEG-45/ Dodecyl Glycol Copolymer | 0,900 |
| Polyglyceryl-3-Diisostearat | 1,800 |
| Cetylstearylisononanoat | 15,500 |
| C₂₀₋₄₀-Alkylstearat | 14,500 |
| Dipropylenglycol | 1,800 |
| 2-Butyloctansäure | 1.000 |
| Parfum, Konservierungsmittel | q.s. |
| Wasser | ad 100,000 |

### Beispiel 9

| Deo-Stift mit hohem Wassergehalt | |
|---|---|
| | Gew.-% |
| PEG-45/ Dodecyl Glycol Copolymer | 0,900 |
| Polyglyceryl-3-Diisostearat | 1,800 |
| Butyloctansäure-2-butyloctanoat | 15,500 |
| C₂₀₋₄₀-Alkylstearat | 14,500 |
| Dipropylenglycol | 1,800 |
| Wollwachssäure | 2.000 |
| Parfum, Konservierungsmittel | q.s. |
| Wasser | ad 100,000 |

### Beispiel 10

| Deo/AT-Stift mit hohem Wassergehalt | |
|---|---|
| | Gew.-% |
| PEG-45/ Dodecyl Glycol Copolymer | 0,900 |
| Polyglyceryl-3-Diisostearat | 1,800 |
| Cetylstearylisononanoat | 6,000 |
| Tridecylisononanoat | 6,000 |
| C₂₀₋₄₀-Alkylstearat | 10,200 |
| Dipropylenglycol | 2,000 |
| Aluminiumchlorohydrat | 10,000 |
| Parfum, Konservierungsmittel | q.s. |
| Wasser | ad 100,000 |

### Beispiel 11

| Deo/AT-Stift mit hohem Wassergehalt | |
|---|---|
| | Gew.-% |
| PEG-45/ Dodecyl Glycol Copolymer | 0,900 |
| Polyglyceryl-3-Diisostearat | 1,800 |
| Capryl-/Caprinsäuretriglycerid | 4,000 |
| Octyldodecanol | 4,000 |
| Dicaprylylether | 4,000 |
| C₂₀₋₄₀-Alkylstearat | 10,000 |
| Stearoxytrimethylsilan und Stearylalkohol | 2,000 |
| Glycerin | 2,000 |
| Aluminiumchlorohydrat | 10,000 |
| Parfum, Konservierungsmittel | q.s. |
| Wasser | ad 100,000 |

### Beispiel 12

| Deo/AT-Stift mit hohem Wassergehalt | |
|---|---|
| | Gew.-% |
| PEG-45/ Dodecyl Glycol Copolymer | 0,900 |
| Polyglyceryl-3-Diisostearat | 1,800 |
| Capryl-/Caprinsäuretriglycerid | 4,000 |
| Octyldodecanol | 4,000 |
| Dicaprylylether | 4,000 |
| C₂₀₋₄₀-Alkylstearat | 12,000 |
| Dimethicon und Trimethylsiloxysilicat | 1,000 |
| Glycerin | 2,000 |
| Aluminiumchlorohydrat | 10,000 |
| Parfum, Konservierungsmittel | q.s. |
| Wasser | ad 100,000 |

### Beispiel 13

| Deo/AT-Stift mit hohem Wassergehalt | |
|---|---|
| | Gew.-% |
| PEG-45/ Dodecyl Glycol Copolymer | 0,900 |
| Polyglyceryl-3-Diisosterat | 1,800 |
| Capryl-/Caprinsäuretriglycerid | 4,000 |
| Octyldodecanol | 4,000 |
| Octacosanylstearat | 6,000 |
| Dicaprylylether | 4,000 |
| Cetylstearylbehenat | 6,000 |
| Glycerin | 2,000 |
| Aluminiumchlorhydrat | 20,000 |
| Parfum, Konservierungsmittel | q.s. |
| Wasser | ad 100,000 |

### Beispiel 14

| Stift mit hohem Wassergehalt | |
|---|---|
| | Gew.-% |
| PEG-45/ Dodecyl Glycol Copolymer | 0,800 |
| Polyglyceryl-3-Diisostearat | 1,600 |
| Butyloctansäure-2-butyloctanoat | 4,000 |
| Capryl-/Caprinsäuretriglycerid | 4,000 |
| Octacosanylstearat | 6,000 |
| Dicaprylylether | 4,000 |
| Cetylstearylbehenat | 6,000 |
| Glycerin | 2,000 |
| Parfum, Konservierungsmittel | q.s. |
| Wasser | ad 100,000 |

### Beispiel 15

| Deo-Stift mit hohem Wassergehalt | |
|---|---|
| | Gew.-% |
| Polyglyceryl-3-Diisostearat | 2,581 |
| Wollwachssäure | 0,860 |
| C₁₂₋₁₅-Alkylbenzoat | 32,687 |
| C₂₀₋₄₀-Alkylstearat | 8,602 |
| Dipropylenglycol | 2,581 |
| Stearylstearat | 12,903 |
| Glycerylcaprylat | 0,817 |
| Parfum, Konservierungsmittel | q.s. |
| Wasser | ad 100,000 |

### Beispiel 16

| Deo-Stift mit hohem Wassergehalt | |
|---|---|
| | Gew.-% |
| Polyglyceryl-3- Diisostearat | 2,581 |
| Wollwachssäure | 0,860 |
| Triisostearin | 32,687 |
| C₂₀₋₄₀-Alkylstearat | 8,602 |
| PEG-40 Hydriertes Rizinusöl | 1,795 |
| Dipropylenglycol | 2,581 |
| Stearylstearat | 12,903 |
| Glycerylcaprylat | 0,817 |
| Parfum, Konservierungsmittel | q.s. |
| Wasser | ad 100,000 |

### Beispiel 17

| Deo-Stift mit hohem Wassergehalt | |
|---|---|
| | Gew.-% |
| Polyglyceryl-3-Diisostearat | 3,834 |
| Wollwachssäure | 1,278 |
| C₂₀₋₄₀-Alkylstearat | 12,779 |
| PEG-40 Hydriertes Rizinusöl | 2,667 |
| Dipropylenglycol | 3,834 |
| Glycerylcaprylat | 1,214 |
| Parfum, Konservierungsmittel | q.s. |
| Wasser | ad 100,000 |

### Beispiel 18

| Deo-Stift mit hohem Wassergehalt | |
|---|---|
| | Gew.-% |
| Polyglyceryl-3-Diisostearat | 2,110 |
| Wollwachssäure | 0,703 |
| Octylisostearat | 15,711 |
| C₂₀₋₄₀-Alkylstearat | 18,759 |
| PEG-40 Hydriertes Rizinusöl | 1,468 |
| Cyclomethicon | 1,172 |
| Dipropylenglycol | 2,110 |
| Glycerylcaprylat | 0,668 |
| Parfum, Konservierungsmittel | q.s. |
| Wasser | ad 100,000 |

### Beispiel 19

| Deo/AT-Stift mit hohem Wassergehalt | |
|---|---|
| | Gew.-% |
| Polyglyceryl-3-Diisostearat | 1,899 |
| Wollwachssäure | 0,633 |
| Octylisostearat | 21,103 |
| C₂₀₋₄₀-Alkylstearat | 16,672 |
| PEG-40 Hydriertes Rizinusöl | 1,321 |
| Dipropylenglycol | 1,899 |
| Aluminiumchlorohydrat | 10,003 |
| Glycerylcaprylat | 0,601 |
| Parfum | 0,760 |
| Methylparaben | 0,158 |
| Wasser | 44,950 |
| Summe: | 100,000 |

### Beispiel 20

| Deo/AT-Stift mit hohem Wassergehalt | |
|---|---|
| | Gew.-% |
| PEG-45/ Dodecyl Glycol Copolymer | 0,800 |
| Sorbitanisostearat | 1,600 |
| Capryl-/Caprinsäuretriglycerid | 4,000 |
| Octyldodecanol | 4,000 |
| Dicaprylylether | 4,000 |
| C₂₀₋₄₀-Alkylstearate | 12,000 |
| Glycerin | 2,000 |
| Aluminiumchlorhydrat | 10,000 |
| Wasser | ad 100,000 |

### Beispiel 21

| Deo/AT-Stift mit hohem Wassergehalt | |
|---|---|
| | Gew.-% |
| PEG-45/ Dodecyl Glycol Copolymer | 0,800 |
| Pentaerythrithylisostearat | 1,600 |
| Capryl-/Caprinsäuretriglycerid | 4,000 |
| Octyldodecanol | 4,000 |
| Dicaprylylether | 4,000 |
| C₂₀₋₄₀-Alkylstearate | 12,000 |
| Glycerin | 2,000 |
| Aluminiumchlorhydrat | 10,000 |
| Wasser | ad 100,000 |

### Beispiel 22

| Deo/AT-Stift mit hohem Wassergehalt | |
|---|---|
| | Gew.-% |
| PEG-45/ Dodecyl Glycol Copolymer | 0,800 |
| PEG-7 Hydrogeniertes Ricinusöl | 1,600 |
| Capryl-/Caprinsäuretriglycerid | 4,000 |
| Octyldodecanol | 4,000 |
| Dicaprylylether | 4,000 |
| C₂₀₋₄₀-Alkylstearate | 12,000 |
| Glycerin | 2,000 |
| Aluminiumchlorhydrat | 10,000 |
| Wasser | ad 100,000 |

### Beispiel 23

| Deo/AT-Stift mit hohem Wassergehalt | |
|---|---|
| | Gew.-% |
| PEG-45/ Dodecyl Glycol Copolymer | 0,800 |
| Sorbitanoleat+PEG-2-hydrogeniertes Ricinus- | 1,600 |
| öl + Ozokerit + hydrogeniertes Ricinusöl | |
| Capryl-/Caprinsäuretriglycerid | 4,000 |
| Octyldodecanol | 4,000 |
| Dicaprylylether | 4,000 |
| C₂₀₋₄₀-Alkylstearate | 12,000 |
| Glycerin | 2,000 |
| Aluminiumchlorhydrat | 10,000 |
| Wasser | ad 100,000 |

### Beispiel 24

| Deo/AT-Stift mit hohem Wassergehalt | |
|---|---|
| | Gew.-% |
| Glyceryllanolat | 1,600 |
| PEG-45/ Dodecyl Glycol Copolymer | 0,800 |
| Capryl-/Caprinsäuretriglycerid | 4,000 |
| Octyldodecanol | 4,000 |
| Dicaprylylether | 4,000 |
| C₂₀₋₄₀-Alkylstearate | 12,000 |
| Glycerin | 2,000 |
| Aluminiumchlorhydrat | 10,000 |
| Wasser | ad 100,000 |

### Beispiel 25

| Deo/AT-Stift mit hohem Wassergehalt | |
|---|---|
| | Gew.-% |
| PEG-45/ Dodecyl Glycol Copolymer | 0,800 |
| Polyglyceryl-2 Dipolyhydroxystearat | 1,600 |
| Capryl-/Caprinsäuretriglycerid | 4,000 |
| Octyldodecanol | 4,000 |
| Dicaprylylether | 4,000 |
| C₂₀₋₄₀-Alkylstearate | 12,000 |
| Glycerin | 2,000 |
| Aluminiumchlorhydrat | 10,000 |
| Wasser | ad 100,000 |

### Beispiel 26

| Stift mit hohem Wassergehalt | |
|---|---|
| | Gew.-% |
| PEG-45/ Dodecyl Glycol Copolymer | 0,800 |
| PEG-40 Sorbitanperisostearat | 1,600 |
| Capryl-/Caprinsäuretriglycerid | 4,000 |
| Octyldodecanol | 4,000 |
| Dicaprylylether | 4,000 |
| C₂₀₋₄₀-Alkylstearate | 12,000 |
| Glycerin | 2,000 |
| Aluminiumchlorhydrat | 10,000 |
| Wasser | ad 100,000 |

### Beispiel 27

| Stift mit hohem Wassergehalt | |
|---|---|
| | Gew.-% |
| PEG-45/ Dodecyl Glycol Copolymer | 0,800 |
| Isostearyldiglycerinsuccinat | 1,600 |
| Capryl-/Caprinsäuretriglycerid | 4,000 |
| Octyldodecanol | 4,000 |
| Dicaprylylether | 4,000 |
| C₂₀₋₄₀-Alkylstearate | 12,000 |
| Glycerin | 2,000 |
| Aluminiumchlorhydrat | 10,000 |
| Wasser | ad 100,000 |

### Beispiel 28

| Stift mit hohem Wassergehalt | |
|---|---|
| | Gew.-% |
| PEG-45/ Dodecyl Glycol Copolymer | 0,800 |
| PEG-5 Cholesterylether | 1,600 |
| Capryl-/Caprinsäuretriglycerid | 4,000 |
| Octyldodecanol | 4,000 |
| Dicaprylylether | 4,000 |
| C₂₀₋₄₀-Alkylstearate | 12,000 |
| Glycerin | 2,000 |
| Aluminiumchlorhydrat | 10,000 |
| Wasser | ad 100,000 |

### Beispiel 29

| Stift mit hohem Wassergehalt | |
|---|---|
| | Gew.-% |
| PEG-45/ Dodecyl Glycol Copolymer | 0,800 |
| Glycerin Isostearat | 1,600 |
| Capryl-/Caprinsäuretriglycerid | 4,000 |
| Octyldodecanol | 4,000 |
| Dicaprylylether | 4,000 |
| C₂₀₋₄₀-Alkylstearate | 12,000 |
| Glycerin | 2,000 |
| Aluminiumchlorhydrat | 10,000 |
| Wasser | ad 100,000 |

### Beispiel 30

| Stift mit hohem Wassergehalt | |
|---|---|
| | Gew.-% |
| PEG-45/ Dodecyl Glycol Copolymer | 0,800 |
| Cetylalkohol | 1,600 |
| Capryl-/Caprinsäuretriglycerid | 4,000 |
| Octyldodecanol | 4,000 |
| Dicaprylylether | 4,000 |
| C₂₀₋₄₀-Alkylstearate | 12,000 |
| Glycerin | 2,000 |
| Aluminiumchlorhydrat | 10,000 |
| Wasser | ad 100,000 |

### Beispiel 31

| Stift mit hohem Wassergehalt | |
|---|---|
| | Gew.-% |
| PEG-45/ Dodecyl Glycol Copolymer | 0,800 |
| Propylenglycoldiisostearat | 1,600 |
| Capryl-/Caprinsäuretriglycerid | 4,000 |
| Octyldodecanol | 4,000 |
| Dicaprylylether | 4,000 |
| C₂₀₋₄₀-Alkylstearate | 12,000 |
| Glycerin | 2,000 |
| Aluminiumchlorhydrat | 10,000 |
| Wasser | ad 100,000 |

### Beispiel 32

| Stift mit hohem Wassergehalt | |
|---|---|
| | Gew.-% |
| PEG-45/ Dodecyl Glycol Copolymer | 0,800 |
| Isostearyl Glycerinether | 1,600 |
| Capryl-/Caprinsäuretriglycerid | 4,000 |
| Octyldodecanol | 4,000 |
| Dicaprylylether | 4,000 |
| C₂₀₋₄₀-Alkylstearate | 12,000 |
| Glycerin | 2,000 |
| Aluminiumchlorhydrat | 10,000 |
| Wasser | ad 100,000 |

### Beispiel 33

| Stift mit hohem Wassergehalt | |
|---|---|
| | Gew.-% |
| PEG-45/ Dodecyl Glycol Copolymer | 0,800 |
| Polyglyceryl-4 Isostearate | 1,600 |
| Capryl-/Caprinsäuretriglycerid | 4,000 |
| Octyldodecanol | 4,000 |
| Dicaprylylether | 4,000 |
| C₂₀₋₄₀-Alkylstearate | 12,000 |
| Glycerin | 2,000 |
| Aluminiumchlorhydrat | 10,000 |
| Wasser | ad 100,000 |

### Beispiel 34

| Stift mit hohem Wassergehalt | |
|---|---|
| | Gew.-% |
| PEG-45/ Dodecyl Glycol Copolymer | 0,800 |
| Glycerinsorbitanisostearat | 1,600 |
| Capryl-/Caprinsäuretriglycerid | 4,000 |
| Octyldodecanol | 4,000 |
| Dicaprylylether | 4,000 |
| C₂₀₋₄₀-Alkylstearate | 12,000 |
| Glycerin | 2,000 |
| Aluminiumchlorhydrat | 10,000 |
| Wasser | ad 100,000 |

### Beispiel 35

| Stift mit hohem Wassergehalt | |
|---|---|
| | Gew.-% |
| PEG-45/ Dodecyl Glycol Copolymer | 0,800 |
| Polyglyceryl-3-Disostearat | 1,600 |
| Capryl-/Caprinsäuretriglycerid | 4,000 |
| Octyldodecanol | 4,000 |
| Dicaprylylether | 4,000 |
| Octacosanylstearat | 12,000 |
| Reiswachs | 2,000 |
| Cetylstearylbehenat | 6.000 |
| Aluminiumchlorhydrat | 10,000 |
| Wasser | ad 100,000 |

### Beispiel 36

| Deo/AT-Stift mit hohem Wassergehalt | |
|---|---|
| | Gew.-% |
| PEG-45/ Dodecyl Glycol Copolymer | 0,900 |
| Polyglyceryl-3-Disostearat | 1,800 |
| Cetylstearylisononanoat | 7,500 |
| C₂₀₋₄₀-Alkylstearat | 14,500 |
| Cyclomethicon | 7,500 |
| Dipropylenglycol | 1,800 |
| Aluminiumchlorhydrat | 20.000 |
| Parfum, Konservierungsmittel | q.s. |
| Wasser | ad 100,000 |

### Beispiel 37

| Deo/AT-Stift mit hohem Wassergehalt Deo/AT-Stift mit hohem Wassergehalt | |
|---|---|
| | Gew.-% |
| Methoxy PEG-22/ Dodecyl Glycol Copolymer | 2.400 |
| Capryl-/Caprinsäuretrigtycerid | 3,000 |
| Octyldodecanol | 4,000 |
| Dicaprylylether | 4,000 |
| C₂₀₋₄₀-Alkylstearate | 12,000 |
| Butandiol | 4,000 |
| Aluminiumchlorhydrat | 20,000 |
| Wasser | ad 100,000 |

### Beispiel 38

| Stift mit hohem Wassergehalt | |
|---|---|
| | Gew.-% |
| PEG-45/ Dodecyl Glycol Copolymer | 0,800 |
| Polyglyceryl-3-Disostearat | 1,600 |
| Capryl-/Caprinsäuretriglycerid | 4,000 |
| Octyldodecanol | 4,000 |
| Dicaprylylether | 4,000 |
| Octacosanylstearat | 12,000 |
| Orangenwachs | 2,000 |
| Cetylstearylbehenat | 6.000 |
| Aluminiumchlorhydrat | 10,000 |
| Wasser | ad 100,000 |

### Beispiel 39

| Stift mit hohem Wassergehalt | |
|---|---|
| | Gew.-% |
| PEG-45/ Dodecyl Glycol Copolymer | 0,800 |
| Polyglyceryt-3-Disostearat | 1,600 |
| Capryl-/Caprinsäuretriglycerid | 4,000 |
| Octyldodecanol | 4,000 |
| Dicaprylylether | 4,000 |
| Octacosanylstearat | 12,000 |
| Zitronenwachs | 2,000 |
| Cetylstearyl Behenat | 6.000 |
| Aluminiumchlorhydrat | 10,000 |
| Wasser | ad 100,000 |

### Beispiel 40

| Stift mit hohem Wassergehalt | |
|---|---|
| | Gew.-% |
| PEG-45/ Dodecyl Glycol Copolymer | 0,800 |
| Polyglyceryl-3-Disostearat | 1,600 |
| Capryl-/Caprinsäuretriglycerid | 4,000 |
| Octyldodecanol | 4,000 |
| Dicaprylylether | 4,000 |
| Octacosanylstearat | 12,000 |
| Grapefruitwachs | 2,000 |
| Cetylstearylbehenat | 6.000 |
| Aluminiumchlorhydrat | 10,000 |
| Wasser | ad 100,000 |

## Patentansprüche

1. Antitranspirant- und/oder Deodorant-Stifte, **dadurch gekennzeichnet, daß** sie
(a) eine Fettphase , welche
(a1) mindestens eine Ölkomponente
(a2) mindestens eine Wachskomponente
(a3) gegebenenfalls weitere in der Fettphase lösliche oder dispergierbare Substanzen
umfaßt:
(b) eine Wasserphase, welche
(b1) 30 bis 85 Gew.% Wasser bezogen auf das Gesamtgewicht der Stiftfüllgut-Masse sowie
(b2) gewünschtenfalls in Wasser lösliche oder dispergierbare Substanzen umfaßt,
c) mindestens eine als Antitranspirant und/oder als Deodorant wirkende Substanz in wirksamer Konzentration,
d) mindestens einen W/O-Emulgator oder ein Gemisch aus mehreren W/O-Emulgatoren,
(e) eine oder mehrere Stabilisatoren, gewählt aus der Gruppe der Substanzen der allgemeinen Struktur A-B-A', wobei A und A' gleiche oder verschiedene hydrophobe organische Reste darstellen und B eine hydrophile Gruppe bedeutet,
(f) gewünschtenfalls weitere grenzflächenaktive Substanzen als Coemulgatoren enthalten, ferner gewünschtenfalls weitere Stabilisatoren und weitere übliche kosmetische und/oder pharmazeutische Hilfs-, Wirk- und/oder Zusatzstoffe, enthalten,

2. Antitranspirant- und/oder Deodorant-Stifte nach Anspruch 1, **dadurch gekennzeichnet, daß** der W/O-Emulgator oder die W/O-Emulgatoren gewählt werden aus der Gruppe der Substanzen der allgemeinen Formel 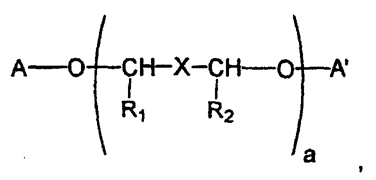 wobei
- A und A' gleiche oder verschiedene hydrophobe organische Reste darstellen,
- a eine Zahl von 1 bis 100, vorzugsweise 2 bis 60, insbesondere 5 bis 40 darstellt,
- X eine Einfachbindung oder die Gruppe 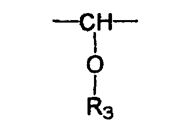
- darstellt,
- R₁ und R₂ unabhängig voneinander aus der Gruppe H, Methyl gewählt werden, daß aber nicht beide Reste gleichzeitig Methyl darstellen,
- R₃ gewählt wird aus der Gruppe H, sowie der verzweigten und unverzweigten, gesättigten und ungesättigten Alkyl- und Acylreste mit 1 - 20 Kohlenstoffatomen,
oder daß der oder die W/O-Emulgatoren gewählt werden aus der Gruppe der Fettalkohole mit 8 - 30 Kohlenstoffatomen, Monoglycerinester gesättigter und/oder ungesättigter, verzweigter und/oder unverzweigter Alkancarbonsäuren einer Kettenlänge von 8 - 24, insbesondere 12 - 18 C-Atomen, Diglycerinester gesättigter und/oder ungesättigter, verzweigter und/oder unverzweigter Alkancarbonsäuren einer Kettenlänge von 8 - 24, insbesondere 12 - 18 C-Atomen, Triglycerinester gesättigter und/oder ungesättigter, verzweigter und/oder unverzweigter Alkancarbonsäuren einer Kettenlänge von 8 - 24, insbesondere 12 - 18 C-Atomen, Polyglycerinester gesättigter und/oder ungesättigter, verzweigter und/oder unverzweigter Alkancarbonsäuren einer Kettenlänge von 8 - 24, insbesondere 12 - 18 C-Atomen mit bis zu 10 Glycerineinheiten, Monoglycerinether gesättigter und/oder ungesättigter, verzweigter und/oder unverzweigter Alkohole einer Kettenlänge von 8 - 24, insbesondere 12 - 18 C-Atomen, Diglycerinether gesättigter und/oder ungesättigter, verzweigter und/oder unverzweigter Alkohole einer Kettenlänge von 8 - 24, insbesondere 12 - 18 C-Atomen, Triglycerinether gesättigter und/oder ungesättigter, verzweigter und/oder unverzweigter Alkohole einer Kettenlänge von 8 - 24, insbesondere 12 - 18 C-Atomen, Polyglycerinether gesättigter und/oder ungesättigter, verzweigter und/oder unverzweigter Alkancarbonsäuren einer Kettenlänge von 8 - 24, insbesondere 12 - 18 C-Atomen mit bis zu 10 Glycerineinheiten, Propylenglycolester gesättigter und/oder ungesättigter, verzweigter und/oder unverzweigter Alkancarbonsäuren einer Kettenlänge von 8 - 24, insbesondere 12 - 18 C-Atomen, Sorbitanester gesättigter und/oder ungesättigter, verzweigter und/oder unverzweigter Alkancarbonsäuren einer Kettenlänge von 8 - 24, insbesondere 12 - 18 C-Atomen, Sorbitanester von Polyolen, insbesondere des Glycerins, Pentaerythritylester gesättigter und/oder ungesättigter, verzweigter und/oder unverzweigter Alkancarbonsäuren einer Kettenlänge von 8 - 24, insbesondere 12 - 18 C-Atomen, Methylglucose Ester gesättigter und/oder ungesättigter, verzweigter und/oder unverzweigter Alkancarbonsäuren einer Kettenlänge von 8 - 24, insbesondere 12 - 18 C-Atomen, Polyglycerin Methylglucose Ester gesättigter und/oder ungesättigter, verzweigter und/oder unverzweigter Alkancarbonsäuren einer Kettenlänge von 8 - 24, insbesondere 12 - 18 C-Atomen,
oder daß die vorstehend genannten Typen von W/O-Emulgatoren zusätzlich in der Weise polyethoxyliert und/oder polypropoxyliert sind, daß sie ethoxylierte und/oder propopoxylierte W/O-Emulgatoren darstellen.

3. Antitranspirant- und/oder Deodorant-Stifte nach Anspruch 2, **dadurch gekennzeichnet, daß** der W/O-Emulgator oder die W/O-Emulgatoren so gewählt werden, daß die Reste A und A' gewählt werden aus der Gruppe der verzweigten und unverzweigten, gesättigten und ungesättigten Alkyl- und Acylreste und Hydroxyacyireste mit 10 - 30 Kohlenstoffatomen sowie femer aus der Gruppe der über Esterfernktionen miteinander verbundenen Hydroxyacylgruppen, nach dem Schema 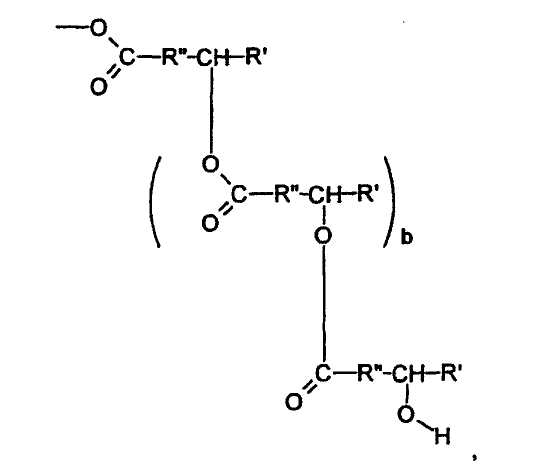 wobei R' gewählt wird aus der Gruppe der verzweigten und unverzweigten Alkylgruppen mit 1 bis 20 Kohlenstoffatomen und R" gewählt wird aus der Gruppe der verzweigten und unverzweigten Alkylengruppen mit 1 bis 20 Kohlenstoffatomen und b Zahlen von 0 bis 200 annehmen kann.

4. Antitranspirant- und/oder Deodorant-Stifte nach Anspruch 1, **dadurch gekennzeichnet, daß** der W/O-Emulgator oder die W/O-Emulgatoren gewählt werden aus der Gruppe PEG-30 Dipolyhydroxystearat, Decaglycerylheptaoleat, Polyglyceryl-3-Diisostearat, PEG-8 Distearat, Diglycerin Dipolyhydroxystearat.

5. Antitranspirant- und/oder Deodorant-Stifte nach Anspruch 1, **dadurch gekennzeichnet, daß** der oder die Stabilisatoren gewählt wird aus der Gruppe der Substanzen der allgemeinen Formel 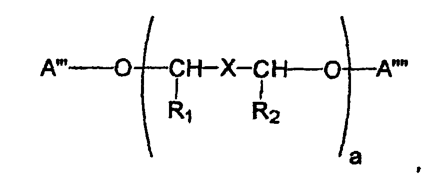 wobei
- A" und A"" gleiche oder verschiedene hydrophobe organische Reste darstellen,
- a eine Zahl von 1 bis 100, vorzugsweise 2 bis 60, darstellt,
- X eine Einfachbindung oder die Gruppe 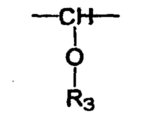 darstellt,
- R₁ und R₂ unabhängig voneinander aus der Gruppe H, Methyl gewählt werden, daß aber nicht beide Reste gleichzeitig Methyl darstellen,
- R₃ gewählt wird aus der Gruppe H, sowie der verzweigten und unverzweigten, gesättigten und ungesättigten Alkyl- und Acylreste mit 1 - 20 Kohlenstoffatomen,
- wobei die Reste A"' und A"" können gleich oder verschieden sein und gewählt werden aus der Gruppe 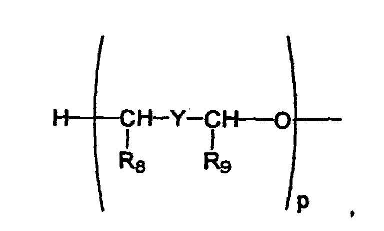
- wobei R₈ und R₉ gleich oder verschieden sein können und gewählt werden aus der Gruppe der gesättigten und ungesättigten Alkyl- und Acylreste mit 1 - 30 Kohlenstoffatomen, p eine Zahl von 1 - 20 darstellt und Y eine Einfachbindung oder die Gruppe 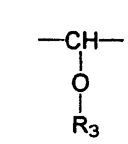 darstellt,
- wobei R₃ gewählt wird aus der Gruppe H, sowie der verzweigten und unverzweigten, gesättigten und ungesättigten Alkyl- und Acylreste mit 1- 30 Kohlenstoffatomen.

6. Antitranspirant- und/oder Deodorant-Stifte nach Anspruch 5, **dadurch gekennzeichnet, daß** als Stabilisator das PEG-45 /Dodecylglycolcopolymer und/oder das PEG-22 / Dodecylglycolcopolymer und/oder das Methoxy PEG-22/Dodecyl Glycol Copolymer verwendet werden.

7. Antitranspirant- und/oder Deodorant-Stifte nach Anspruch 1, **dadurch gekennzeichnet, daß** die Ölkomponente oder die Gesamtheit der Ölkomponenten der erfindungsgemäßen wasserreichen Stifte gewählt wird aus der Gruppe der Ester aus gesättigten und/oder ungesättigten, verzweigten und/oder unverzweigten Alkancarbonsäuren einer Kettenlänge von 14 bis 44 C-Atomen und gesättigten und/oder ungesättigten, verzweigten und/oder unverzweigten Alkoholen einer Kettenlänge von 14 bis 44 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen sofern die Ölkomponente oder die Gesamtheit der Ölkomponenten bei Raumtemperatur eine Flüssigkeit darstellen.

8. Antitranspirant- und/oder Deodorant-Stifte nach Anspruch 1, **dadurch gekennzeichnet, daß** die Ölkomponente oder die Gesamtheit der Ölkomponenten der erfindungsgemäßen wasserreichen Stifte gewählt wird aus der Gruppe der verzweigten und unverzweigten Kohlenwasserstoffe, der cyclischen oder linearen Silikonöle, der Dialkylether, der Gruppe der gesättigten oder ungesättigten, verzweigten Alkohole, sowie der Fettsäuretriglyceride, namentlich der synthetischen oder natürlichen Triglycerinester gesättigter und/oder ungesättigter, verzweigter und/oder unverzweigter Alkancarbonsäuren einer Kettenlänge von 8 bis 24, insbesondere 12 - 18 C-Atomen.

9. Antitranspirant- und/oder Deodorant-Stifte nach Anspruch 1, **dadurch gekennzeichnet, daß** die Wachskomponente oder die Gesamtheit der Wachskomponenten der erfindungsgemäßen W/O-Emulsionsstifte gewählt wird aus der Gruppe der Ester aus gesättigten und/oder ungesättigten, verzweigten und/oder unverzweigten Alkancarbonsäuren einer Kettenlänge von 1 bis 80 C-Atomen und gesättigten und/oder ungesättigten, verzweigten und/oder unverzweigten Alkoholen einer Kettenlänge von 1 bis 80 C-Atomen, aus der Gruppe der Ester aus aromatischen Carbonsäuren und gesättigten und/oder ungesättigten, verzweigten und/oder unverzweigten Alkoholen einer Kettenlänge von 1 bis 80 C-Atomen sofem die Wachskomponente oder die Gesamtheit der Wachskomponenten bei Raumtemperatur einen Festkörper darstellen.

10. Antitranspirant- und/oder Deodorant-Stifte nach Anspruch 1, **dadurch gekennzeichnet, daß** als als Antitranspirant wirkende Substanz das Aluminium-chlorhydrat gewählt wird.

11. Antitranspirant- und/oder Deodorant-Stifte nach Anspruch 1, durch einen zusätzlichen Gehalt an einem oder mehreren wasserlöslichen und/oder mit Wasser quellbaren Polymeren gekennzeichnet, insbesondere mit Alkylgruppen veretherte Cellulose- und/oder Stärkederivate, bevorzugt β-Glucane, Xanthangummi, Dextrane, Hydroxymethylcellulose, Hydroxyethylcellulose und/oder Hydroxypropylcellulose, Methoxy-PEG-22/ Dodecyl-Glycol-Copolymere, Poloxamere, mit einem oder mehreren n-Octenylsuccinatresten veresterter hydrophiler Stärke.

## Claims

1. Antiperspirant and/or deodorant sticks, **characterized in that** they comprise,
(a) a lipid phase, which comprises
(a1) at least one oil component
(a2) at least one wax component
(a3) optionally other substances soluble or dispersible in the lipid phase,
(b) an aqueous phase, which comprises
(b1) from 30 to 85% by weight of water, based on the total weight of the stick composition and
(b2) if desired, substances soluble or dispersible in water,
(c) at least one substance which acts as an antiperspirant and/or as a deodorant, in an effective concentration,
(d) at least one W/O emulsifier or a mixture of two or more W/O emulsifiers,
(e) one or more stabilizers, chosen from the group of substances of the general structure A-B-A', where A and A' are identical or different hydrophobic organic radicals, and B is a hydrophilic group,
(f) if desired, further surface-active substances as coemulsifiers, and also, if desired, further stabilizers and further customary cosmetic and/or pharmaceutical auxiliaries, active ingredients and/or additives.

2. Antiperspirant and/or deodorant sticks according to Claim 1, **characterized in that** the W/O emulsifier or the W/O emulsifiers are chosen from the group of substances of the general formula 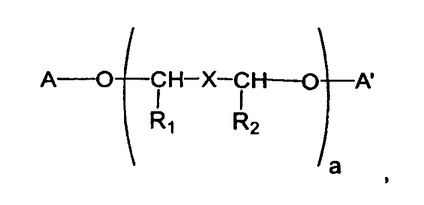 where
- A and A' are identical or different hydrophobic organic radicals,
- a is a number from 1 to 100, preferably from 2 to 60, in particular from 5 to 40,
- X is a single bond or the group 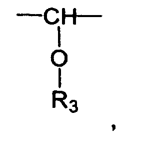
- R₁ and R₂ independently of one another are chosen from the group consisting of H or methyl, but such that the two radicals are not methyl at the same time,
- R₃ is chosen from the group consisting of H and the branched and unbranched, saturated and unsaturated alkyl and acyl radicals having 1 - 20 carbon atoms,
or **in that** the W/O emulsifier(s) is/are chosen from the group of fatty alcohols having 8 - 30 carbon atoms, monoglycerol esters of saturated and/or unsaturated, branched and/or unbranched alkanecarboxylic acids having a chain length of 8 - 24, in particular 12 - 18, carbon atoms, diglycerol esters of saturated and/or unsaturated, branched and/or unbranched alkanecarboxylic acids having a chain length of 8 - 24, in particular 12 - 18, carbon atoms, triglycerol esters of saturated and/or unsaturated, branched and/or unbranched alkanecarboxylic acids having a chain length of 8 - 24, in particular 12 - 18, carbon atoms, polyglycerol esters of saturated and/or unsaturated, branched and/or unbranched alkanecarboxylic acids having a chain length of 8 - 24, in particular 12 - 18, carbon atoms and up to 10 glycerol units, monoglycerol ethers of saturated and/or unsaturated, branched and/or unbranched alcohols having a chain length of 8 - 24, in particular 12 - 18, carbon atoms, diglycerol ethers of saturated and/or unsaturated, branched and/or unbranched alcohols having a chain length of 8 - 24, in particular 12 - 18, carbon atoms, triglycerol ethers of saturated and/or unsaturated, branched and/or unbranched alcohols having a chain length of 8 - 24, in particular 12 - 18, carbon atoms, polyglycerol ethers of saturated and/or unsaturated, branched and/or unbranched alkanecarboxylic acids having a chain length of 8 - 24, in particular 12 - 18, carbon atoms and up to 10 glycerol units, propylene glycol esters of saturated and/or unsaturated, branched and/or unbranched alkanecarboxylic acids having a chain length of 8 - 24, in particular 12 - 18, carbon atoms, sorbitan esters of saturated and/or unsaturated, branched and/or unbranched alkanecarboxylic acids having a chain length of 8 - 24, in particular 12 - 18, carbon atoms, sorbitan esters of polyols, in particular of glycerol, pentaerythrityl esters of saturated and/or unsaturated, branched and/or unbranched alkanecarboxylic acids having a chain length of 8 - 24, in particular 12 - 18, carbon atoms, methylglucose esters of saturated and/or unsaturated, branched and/or unbranched alkanecarboxylic acids having a chain length of 8 - 24, in particular 12 - 18, carbon atoms, polyglycerol methylglucose esters of saturated and/or unsaturated, branched and/or unbranched alkanecarboxylic acids having a chain length of 8 - 24, in particular 12 - 18, carbon atoms,
or **in that** the abovementioned types of W/O emulsifiers are additionally polyethoxylated and/or polypropoxylated such that they are ethoxylated and/or propoxylated W/O emulsifers.

3. Antiperspirant and/or deodorant sticks according to Claim 2, **characterized in that** the W/O emulsifier or the W/O emulsifiers is/are chosen such that the radicals A and A' are advantageously chosen from the group of branched and unbranched, saturated and unsaturated alkyl and acyl radicals and hydroxyacyl radicals having 10 - 30 carbon atoms and also from the group of hydroxyacyl groups joined together via ester functions, according to the formula 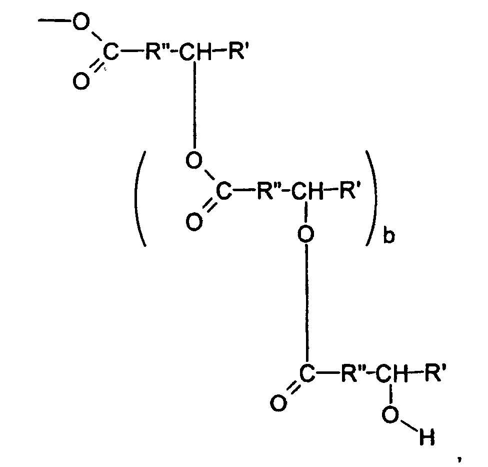 where R' is chosen from the group of branched and unbranched alkyl groups having from 1 to 20 carbon atoms, and R" is chosen from the group of branched and unbranched alkylene groups having from 1 to 20 carbon atoms, and b can assume numbers from 0 to 200.

4. Antiperspirant and/or deodorant sticks according to Claim 1, **characterized in that** the W/O emulsifier or the W/O emulsifiers is/are chosen from the group consisting of PEG-30 dipolyhydroxystearate, decaglyceryl heptaoleate, polyglyceryl-3 diisostearate, PEG-8 distearate, diglycerol dipolyhydroxystearate.

5. Antiperspirant and/or deodorant sticks according to Claim 1, **characterized in that** the stabilizer(s) is/are chosen from the group of substances of the general formula 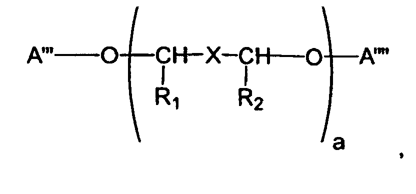 where
- A"' and A"" are identical or different hydrophobic organic radicals,
- a is a number from 1 to 100, preferably from 2 to 60,
- X is a single bond or the group 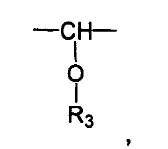
- R₁ and R₂ independently of one another are chosen from the group consisting of H and methyl, but such that the two radicals are not methyl at the same time,
- R₃ is chosen from the group consisting of H and the branched and unbranched, saturated and unsaturated alkyl and acyl radicals having 1 - 20 carbon atoms,
- where the radicals A''' and A"" can be identical or different and are chosen from the group 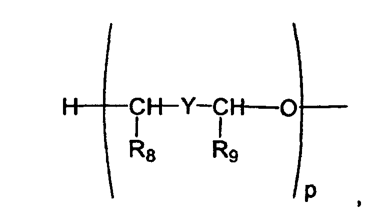
- where R₈ and R₉ can be identical or different and are chosen from the group of saturated and unsaturated alkyl and acyl radicals having 1 - 30 carbon atoms, p is a number from 1 to 20, and Y is a single bond or the group 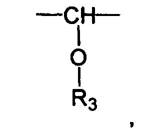
- where R₃ is chosen from the group consisting of H and the branched and unbranched, saturated and unsaturated alkyl and acyl radicals having 1 - 30 carbon atoms.

6. Antiperspirant and/or deodorant sticks according to Claim 5, **characterized in that** the stabilizer used is the PEG-45/dodecyl glycol copolymer and/or the PEG-22/dodecyl glycol copolymer and/or the methoxy-PEG-22/dodecyl glycol copolymer.

7. Antiperspirant and/or deodorant sticks according to Claim 1, **characterized in that** the oil component or the totality of the oil components of the novel water-rich sticks is chosen from the group of esters of saturated and/or unsaturated, branched and/or unbranched alkanecarboxylic acids having a chain length of from 14 to 44 carbon atoms and saturated and/or unsaturated, branched and/or unbranched alcohols having a chain length of from 14 to 44 carbon atoms, from the group of esters of aromatic carboxylic acids and saturated and/or unsaturated, branched and/or unbranched alcohols having a chain length of from 3 to 30 carbon atoms, provided the oil component or the totality of the oil components is a liquid at room temperature.

8. Antiperspirant and/or deodorant sticks according to Claim 1, **characterized in that** the oil component or the totality of the oil components of the novel water-rich sticks is chosen from the group of branched and unbranched hydrocarbons, cyclic or linear silicone oils, dialkyl ethers, the group of saturated or unsaturated, branched alcohols, and the fatty acid triglycerides, namely the synthetic or natural triglycerol esters of saturated and/or unsaturated, branched and/or unbranched alkanecarboxylic acids having a chain length of from 8 to 24, in particular 12 - 18, carbon atoms.

9. Antiperspirant and/or deodorant sticks according to Claim 1, **characterized in that** the wax component or the totality of the wax components of the novel W/O emulsion sticks is chosen from the group of esters of saturated and/or unsaturated, branched and/or unbranched alkanecarboxylic acids having a chain length of from 1 to 80 carbon atoms and saturated and/or unsaturated, branched and/or unbranched alcohols having a chain length of from 1 to 80 carbon atoms, from the group of esters of aromatic carboxylic acids and saturated and/or unsaturated, branched and/or unbranched alcohols having a chain length of from 1 to 80 carbon atoms, provided that the wax component or the totality of the wax components is a solid at room temperature.

10. Antiperspirant and/or deodorant sticks according to Claim 1, **characterized in that** the substance which acts as antiperspirant is aluminium chlorohydrate.

11. Antiperspirant and/or deodorant sticks according to Claim 1, **characterized by** an additional content of one or more water-soluble and/or water-swellable polymers, in particular alkyl-etherified cellulose and/or starch derivatives, preferably β-glucans, xanthan gum, dextrans, hydroxymethylcellulose, hydroxyethylcellulose and/or hydroxypropylcellulose, methoxy PEG-22/ dodecyl glycol copolymers, poloxamers, hydrophilic starch esterified with one or more n-octenylsuccinate radicals.

## Revendications

1. Bâtons antisudoraux et/ou déodorants, **caractérisés en ce qu'**ils comprennent:
a) une phase grasse, comprenant
(a1) au moins un composant huileux,
(a2) au moins un composant cireux,
(a3) éventuellement d'autres substances solubles ou dispersibles dans la phase grasse,
b) une phase aqueuse, comprenant
(b1) de 30 à 85% en poids d'eau, par rapport au poids total de la masse de remplissage du bâton, ainsi que
(b2) éventuellement des substances solubles ou dispersibles dans l'eau,
c) au moins une substance à action antisudorale et/ou déodorante en une concentration efficace,
d) au moins un émulsifiant E/H ou un mélange de plusieurs émulsifiants E/H,
e) un ou plusieurs stabilisants, choisis dans le groupe des substances de la structure générale A-B-A', où A et A' représentent des restes organiques hydrophobes identiques ou différents, et B désigne un groupe hydrophile,
f) éventuellement d'autres substances tensioactives en tant que coémulsifiants, ainsi que, si on le souhaite, d'autres stabilisants et d'autres adjuvants, agents actifs et/ou additifs cosmétiques et/ou pharmaceutiques usuels.

2. Bâtons antisudoraux et/ou déodorants selon la revendication 1, **caractérisés en ce que** l'émulsifiant E/H ou les émulsifiants E/H sont choisis dans le groupe des substances de la formule générale 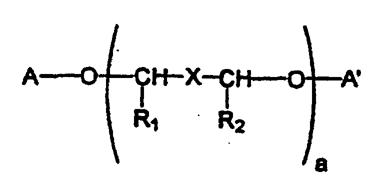 dans laquelle
- A et A' représentent des restes organiques hydrophobes identiques ou différents,
- a est un nombre de 1 à 100, de préférence de 2 à 60, en particulier de 5 à 40,
- X représente une liaison simple ou le groupe 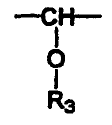
- R₁ et R₂ sont choisis indépendamment l'un de l'autre dans le groupe formé par H et un méthyle, les deux restes ne représentant toutefois pas simultanément un méthyle,
- R₃ est choisi dans le groupe formé par H et les restes alkyle et acyle ramifiés et non ramifiés, saturés et insaturés, comportant de 1 à 20 atomes de carbone,
ou bien que le ou les émulsifiants E/H sont choisis dans le groupe des alcools gras comportant de 8 à 30 atomes de carbone, des esters monoglycériques d'acides alcane-carboxyliques saturés et/ou insaturés, ramifiés et/ou non ramifiés et d'une longueur de chaîne de 8 à 24, en particulier de 12 à 18 atomes de carbone, des esters diglycériques d'acides alcane-carboxyliques saturés et/ou insaturés, ramifiés et/ou non ramifiés et d'une longueur de chaîne de 8 à 24, en particulier de 12 à 18 atomes de C, des esters triglycériques d'acides alcane-carboxyliques saturés et/ou insaturés, ramifiés et/ou non ramifiés et d'une longueur de chaîne de 8 à 24, en particulier de 12 à 18 atomes de C, des esters polyglycériques d'acides alcane-carboxyliques saturés et/ou insaturés, ramifiés et/ou non ramifiés et d'une longueur de chaîne de 8 à 24, en particulier de 12 à 18 atomes de C avec jusqu'à 10 unités de glycérol, des éthers monoglycériques d'alcools saturés et/ou insaturés, ramifiés et/ou non ramifiés et d'une longueur de chaîne de 8 à 24, en particulier de 12 à 18 atomes de C, des éthers diglycériques d'alcools saturés et/ou insaturés, ramifiés et/ou non ramifiés et d'une longueur de chaîne de 8 à 24, en particulier de 12 à 18 atomes de C, des éthers triglycériques d'alcools saturés et/ou insaturés, ramifiés et/ou non ramifiés et d'une longueur de chaîne de 8 à 24, en particulier de 12 à 18 atomes de C, des éthers polyglycériques d'acides alcane-carboxyliques saturés et/ou insaturés, ramifiés et/ou non ramifiés et d'une longueur de chaîne de 8 à 24, en particulier de 12 à 18 atomes de C avec jusqu'à 10 unités de glycérol, des esters de propylèneglycol d'acides alcane-carboxyliques saturés et/ou insaturés, ramifiés et/ou non ramifiés et d'une longueur de chaîne de 8 à 24, en particulier de 12 à 18 atomes de C, des esters de sorbitanne d'acides alcane-carboxyliques saturés et/ou insaturés, ramifiés et/ou non ramifiés et d'une longueur de chaîne de 8 à 24, en particulier de 12 à 18 atomes de C, des esters de sorbitanne de polyols, en particulier du glycérol, des esters de pentaérythrityle d'acides alcane-carboxyliques saturés et/ou insaturés, ramifiés et/ou non ramifiés et d'une longueur de chaîne de 8 à 24, en particulier de 12 à 18 atomes de C, des esters de méthylglucose d'acides alcane-carboxyliques saturés et/ou insaturés, ramifiés et/ou non ramifiés et d'une longueur de chaîne de 8 à 24, en particulier de 12 à 18 atomes de C, des esters de polyglycérol méthylglucose d'acides alcane-carboxyliques saturés et/ou insaturés, ramifiés et/ou non ramifiés et d'une longueur de chaîne de 8 à 24, en particulier de 12 à 18 atomes de C,
ou bien que les types d'émulsifiants E/H précités sont en outre polyéthoxylés et/ou polypropoxylés, de manière à représenter des émulsifiants E/H éthoxylés ou propoxylés.

3. Bâtons antisudoraux et/ou déodorants selon la revendication 2, **caractérisés en ce que** l'émulsifiant E/H ou les émulsifiants E/H sont choisis de manière que les restes A et A' soient choisis dans le groupe des restes alkyle et acyle et des restes hydroxyacyle ramifiés et non ramifiés, saturés et insaturés, comportant de 10 à 30 atomes de carbone, ainsi qu'en outre dans le groupe des groupes hydroxyacyle liés ensemble via des fonctions ester, selon le schéma 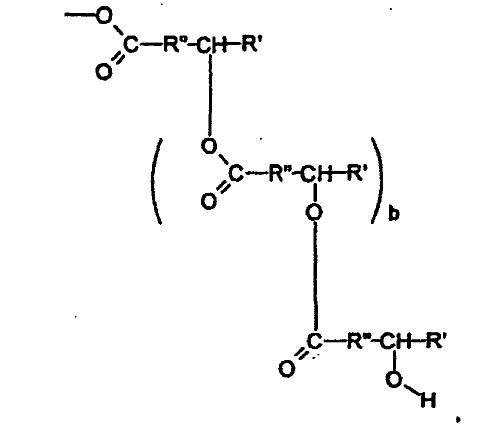 dans lequel R' est choisi dans le groupe des groupes alkyle ramifiés et non ramifiés comportant de 1 à 20 atomes de carbone et R" est choisi dans le groupe des groupes alkylène ramifiés et non ramifiés comportant de 1 à 20 atomes de carbone, et b peut avoir une valeur de 0 à 200.

4. Bâtons antisudoraux et/ou déodorants selon la revendication 1, **caractérisés en ce que** l'émulsifiant E/H ou les émulsifiants E/H sont choisis dans le groupe formé par le dipoly(hydroxystéarate) de PEG-30, l'heptaoléate de décaglycéryle, le 3-diisostéarate de polyglycéryle, le distéarate de PEG-8, le dipoly(hydroxystéarate) de diglycérol.

5. Bâtons antisudoraux et/ou déodorants selon la revendication 1, **caractérisés en ce que** le ou les stabilisants sont choisis dans le groupe des substances 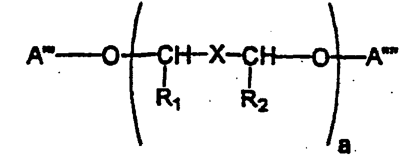 dans laquelle
- A"' et A"" représentent des restes organiques hydrophobes identiques ou différents,
- a est un nombre de 1 à 100, de préférence de 2 à 60,
- X représente une liaison simple ou le groupe 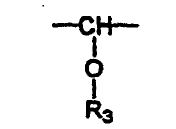
- R₁ et R₂ sont choisis indépendamment l'un de l'autre dans le groupe formé par H et un méthyle, les deux restes ne représentant toutefois pas simultanément un méthyle,
- R₃ est choisi dans le groupe formé par H et les restes alkyle et acyle ramifiés et non ramifiés, saturés et insaturés, comportant de 1 à 20 atomes de carbone,
- les restes A"' et A"" pouvant être identiques ou différents et étant choisis dans le groupe 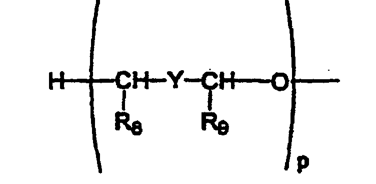
où R₈ et R₉ peuvent être identiques ou différents et sont choisis dans le groupe des restes alkyle et acyle saturés et insaturés comportant de 1 à 30 atomes de carbone, p représente un nombre de 1 à 20 et Y représente une liaison simple ou le groupe 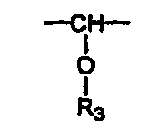 où R₃ est choisi dans le groupe formé par H et les restes alkyle et acyle ramifiés et non ramifiés, saturés et insaturés, comportant de 1 à 30 atomes de carbone.

6. Bâtons antisudoraux et/ou déodorants selon la revendication 5, **caractérisés en ce qu'**on utilise comme stabilisant le copolymère de PEG-45/dodécylglycol et/ou le copolymère de PEG-22/dodécylglycol et/ou le copolymère de méthoxy-PEG-22/dodécylglycol.

7. Bâtons antisudoraux et/ou déodorants selon la revendication 1, **caractérisés en ce que** le composant huileux ou l'ensemble des composants huileux des bâtons à haute teneur en eau selon l'invention sont choisis dans le groupe des esters d'acides alcane-carboxyliques saturés et/ou insaturés, ramifiés et/ou non ramifiés et d'une longueur de chaîne de 14 à 44 atomes de C et d'alcools saturés et/ou insaturés, ramifiés et/ou non ramifiés et d'une longueur de chaîne de 14 à 44 atomes de C, dans le groupe des esters d'acides carboxyliques aromatiques et d'alcools saturés et/ou insaturés, ramifiés et/ou non ramifiés d'une longueur de chaîne de 3 à 30 atomes de C, dans la mesure où le composant huileux ou l'ensemble des composants huileux constituent un liquide à température ambiante.

8. Bâtons antisudoraux et/ou déodorants selon la revendication 1, **caractérisés en ce que** le composant huileux ou l'ensemble des composants huileux des bâtons à haute teneur en eau selon l'invention sont choisis dans le groupe des hydrocarbures ramifiés et non ramifiés, des huiles siliconées cycliques ou linéaires, des éthers dialkyliques, le groupe des alcools saturés ou insaturés, ramifiés, ainsi que des triglycérides d'acides gras, à savoir les esters triglycériques synthétiques ou naturels d'acides alcane-carboxyliques saturés et/ou insaturés, ramifiés et/ou non ramifiés et d'une longueur de chaîne de 8 à 24, en particulier de 12 à 18 atomes de C.

9. Bâtons antisudoraux et/ou déodorants selon la revendication 1, **caractérisés en ce que** le composant cireux ou l'ensemble des composants cireux des bâtons d'émulsions E/H selon l'invention sont choisis dans le groupe des esters d'acides alcane-carboxyliques saturés et/ou insaturés, ramifiés et/ou non ramifiés et d'une longueur de chaîne de 1 à 80 atomes de C et d'alcools saturés et/ou insaturés, ramifiés et/ou non ramifiés et d'une longueur de chaîne de 1 à 80 atomes de C, dans le groupe des esters d'acides carboxyliques aromatiques et d'alcools saturés et/ou insaturés, ramifiés et/ou non ramifiés et d'une longueur de chaîne de 1 à 80 atomes de C dans la mesure où le composant cireux ou l'ensemble des composants cireux constituent un solide à température ambiante.

10. Bâton antisudoral et/ou déodorant selon la revendication 1, **caractérisé en ce que** l'on choisit comme substance à action antisudorale du chlorhydrate d'aluminium.

11. Bâton antisudoral et/ou déodorant selon la revendication 1, **caractérisé par** une teneur additionnelle en un ou plusieurs polymères solubles dans l'eau et/ou gonflables à l'eau, en particulier des dérivés de cellulose et/ou d'amidon éthérifiés par des groupes alkyle, de préférence du β-glucane, de la gomme xanthane, du dextrane, de l'hydroxyméthylcellulose, de l'hydroxyéthylcellulose et/ou de l'hydroxypropylcellulose, des copolymères de méthoxy-PEG-22/dodécylglycol, des poloxamères, avec un ou plusieurs amidons hydrophiles estérifiés avec des restes de n-octénylsuccinate.
